(19) Europäisches Patentamt
European Patent Office
Office européen des brevets

(11) EP 1 964 512 A2

(12) **EUROPEAN PATENT APPLICATION**

(43) Date of publication:
**03.09.2008 Bulletin 2008/36**

(51) Int Cl.:
***A61B 5/00*** *(2006.01)* ***A61B 5/053*** *(2006.01)*

(21) Application number: **08003552.0**

(22) Date of filing: **27.02.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **28.02.2007 JP 2007048262**
**26.03.2007 JP 2007078375**

(71) Applicant: **SYSMEX CORPORATION**
**Kobe-shi,**
**Hyogo 651-0073 (JP)**

(72) Inventors:
- **Hagino, Kei**
  **Kobe-shi**
  **Hyogo 651-0073 (JP)**
- **Okada, Seiki**
  **Kobe-shi**
  **Hyogo 651-0073 (JP)**
- **Maekawa, Yasunori**
  **Kobe-shi**
  **Hyogo 651-0073 (JP)**
- **Asano, Kaoru**
  **Kobe-shi**
  **Hyogo 651-0073 (JP)**

(74) Representative: **HOFFMANN EITLE**
**Patent- und Rechtsanwälte**
**Arabellastrasse 4**
**81925 München (DE)**

(54) **Method of measuring skin conductance, method of analyzing component concentration, skin conductive measuring apparatus, and component concentration analyzer**

(57) The present invention provides a method of measuring skin conductance which can obtain an accuracy measurement result, comprising the steps of: supplying a low conductivity medium as a medium for holding tissue fluid that contains a predetermined component of a subject to a medium containing part; disposing the medium containing part on an extraction region of a skin of the subject; extracting tissue fluid through the extraction region into the low conductivity medium within the medium containing part; supplying electric power between a first electrode for supplying electric power to the extraction region and a second electrode for supplying electric power to the skin outside the extraction region; and measuring the conductance of the extraction region based on the electric power supplied between the first electrode and second electrode. A method of analyzing a component concentration, a skin conductance measuring apparatus, and a component concentration analyzer are also disclosed.

FIG. 1

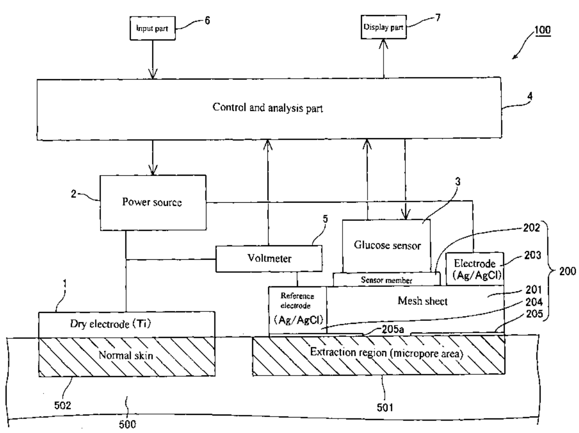


EP 1 964 512 A2

## Description

FIELD OF THE INVENTION

The present invention relates to a method for measuring skin permeability, method for analyzing component concentration, device for measuring skin permeability, and device for analyzing component concentration, and specifically relates to a method for measuring skin permeability which includes a process for supplying a medium for holding tissue fluid, which contains predetermined components of a subject, to a medium containing part, a method for analyzing the component concentrations, a device for measuring skin permeability which is provided with a medium containing part for receiving tissue fluid that contains predetermined components of a subject, and a device for analyzing the component concentrations.

The present invention further relates to a skin conductance measuring device, component concentration analyzing device, skin conductance measuring method, and component concentration analyzing method, and specifically relates to a skin conductance measuring device for measuring the conductance of the skin of a subject, a component concentration analyzing device for analyzing the concentrations of predetermined components contained in the tissue fluid of the subject using the measured skin conductance, a skin conductance measuring method, and a component concentration analyzing method.

BACKGROUND

Blood sugar level analyzing methods are know which are provided with a step of supplying a medium, which holds tissue fluid that includes predetermined components of a subject, to a medium containing part (US20060029991). Component concentration analyzers are also known which measure the conductance of the skin of a subject, and analyze the component concentrations of predetermined components contained in the tissue fluid of the subject using this conductance (US20060029991).

In US20060029991, glucose is extracted through the skin of a subject by so-called reverse iontophoresis for analyzing the blood sugar level without collecting blood. That is, physiological saline solution is supplied as a medium for holding the glucose within the chamber disposed on the skin of the subject, and an electric current is applied to the skin for a predetermined time through electrodes positioned on the skin of the subject. Ions present in the tissue fluid of the subject therefore migrate to the electric potential formed by the electric current. The glucose contained in the tissue fluid also migrates in conjunction with the migration of the ionic substance, and the tissue fluid containing the glucose extracted from the skin is holded in the physiological saline within the chamber. The amount of glucose within the chamber is measured by a sensor, and the glucose extraction speed is calculated by measuring the time during which the electric current was applied to obtain the glucose amount.

In US2006009991, the conductance of the skin of the subject is measured to correct the extraction amount of the transdermally extracted glucose, and the condition of the skin of the subject is monitored by this conductance. Then, the permeability of glucose through the skin (glucose permeability) in correlation with the conductance is estimated from the conductance, and the blood sugar level of the subject is calculated based on the glucose extraction rate and glucose permeability.

In US 20060029991, however, the conductance and glucose permeability may poorly correlate as a result of the salts such as Na+, Cl and the like contained in the physiological saline solution because physiological saline solution is used as the medium for holding the tissue fluid which includes glucose. Estimating glucose permeability with good accuracy is therefore difficult. Since it is difficult to accurately estimate glucose permeability, it is also difficult to accurately calculate the blood sugar level based on the since the calculation is based on the glucose permeability.

The component concentration analyzer disclosed in US20060029991 is provided with a switching circuit and three electrodes including a first electrode, second electrode, and third electrode disposed on the skin of a subject. The switching circuit is configured so as to be capable of switching the current flow pattern among a first current flow pattern in which a direct current flows to the skin for a predetermined time through the first electrode and second electrode, a second current flow pattern in which a direct current flows to the skin for a predetermined time through the second electrode and third electrode, and a third current flow pattern in which a direct current flows to the skin for a predetermined time through the third electrode and first electrode.

In US20060029991, the conductance of the skin is calculated based on a first electrical information, second electrical information, and third electrical information obtained by the respective first current flow pattern second current flow pattern, and third current flow pattern, and calculates the component concentration of a predetermined component contained in the tissue fluid of the subject based on the calculated conductance.

In the component concentration analyzer disclosed in US20060029991, however, a problem arises in that the current flow time is lengthened to calculate the conductance of the skin because the current must flow for predetermined times in the respective first current flow pattern, second current flow pattern, and third current flow pattern in order to obtain the first electrical information, second electrical information, and third electrical information necessary to calculate the conductance of the skin. A further problem arises in that electric power consumption is increased since a direct current is applied for a long time. A further problem is the complexity of the apparatus structure since switching circuits are necessary to switch the three electrodes and current flow patterns.

SUMMARY

The scope of the present invention is defined solely by the appended claims, and is not affected to any degree by the statements within this summary.

To resolve above-mentioned problems, a first object of the present invention is to provide a method of measuring skin permeability capable of estimating, with greater accuracy, the permeability of predetermined components through the skin, and a method of analyzing component concentrations capable of calculating, with greater accuracy, the concentrations of predetermined components in tissue fluid.

A further object of the present invention is to provide a skin permeability measuring apparatus capable of estimating, with greater accuracy, the permeability of predetermined components through the skin, and a component concentration analyzer capable of calculating, with greater accuracy, the concentrations of predetermined components in tissue fluid.

A still further object of the present invention is to provide a method for measuring skin conductance, a method for analyzing component concentrations, skin conductance measuring apparatus, and component concentration analyzer capable of calculating the conductance of skin with a short current application time, low electric power consumption, and simplified apparatus structure.

BRIEF DESCRIPTION OF THE DRAWINGS

FIG. 1 is a block diagram showing the structure of a glucose concentration analyzer of a first embodiment of the present invention;
FIG. 2 is a perspective view showing the micro needles used when forming the micropores in the skin of a subject;
FIG. 3 is a cross section view showing the condition of the skin with the micropores formed by the micro needles shown in FIG. 2;
FIG. 4 is a flow chart illustrating the analyzing operation of the glucose concentration analyzer shown in FIG. 1;
FIG. 5 is a characteristics chart which shows the relationship between conductance and the glucose permeability in the case of the first embodiment;
FIG. 6 is a characteristics chart which shows the relationship between conductance and the glucose permeability in the case of the second embodiment;
FIG. 7 is a characteristics chart which shows the relationship between conductance and the glucose permeability in the case of the third embodiment;
FIG. 8 is a characteristics chart which shows the relationship between conductance and the glucose permeability in the case of the fourth embodiment;
FIG. 9 is a characteristics chart which shows the relationship between conductance and the glucose permeability in the case of the first comparative example;
FIG. 10 is a bar graph showing the glucose permeability measurement errors for the first through fourth embodiments and the first comparative example;
FIG. 11 illustrates a hypothesis resulting from consideration of the mechanism which produces the result of the present invention;
FIG. 12 illustrates a hypothesis resulting from consideration of the mechanism which produces the result of the present invention;
FIG. 13 is a block diagram showing the structure of a glucose concentration analyzer of a second embodiment of the present invention;
FIG. 14 is a flow chart illustrating the measuring operation of the conductance measuring apparatus of the glucose concentration analyzer shown in FIG. 13;
FIG. 15 is a flow chart illustrating the analyzing operation of the concentration analyzer of the glucose concentration analyzer shown in FIG. 13;
FIG. 16 is a block diagram showing the structure of a glucose concentration analyzer of a third embodiment of the present invention;
FIG. 17 is an equivalent circuit illustrating a first current flow pattern of the glucose concentration analyzer shown in FIG. 16;

FIG. 18 is an equivalent circuit illustrating a second current flow pattern of the glucose concentration analyzer shown in FIG. 16;
FIG. 19 is an equivalent circuit illustrating a third current flow pattern of the glucose concentration analyzer shown in FIG. 16;
FIG. 20 is a flow chart illustrating the analyzing operation of the glucose concentration analyzer shown in FIG. 16;
FIG. 21 is an external view showing the puncture tool used when forming the micropores in the skin of a subject;
FIG. 22 is a perspective view of a micro needle chip mounted on the puncture tool shown in FIG. 21;
FIG. 23 is a block diagram showing the structure of a glucose concentration analyzer of a fourth embodiment of the present invention;
FIG. 24 is a flow chart illustrating the analyzing operation of the glucose concentration analyzer shown in FIG. 23;
FIG. 25 is a circuit diagram showing a circuit configuring the equivalent and glucose concentration analyzer of FIG. 23;
FIG. 26 is a graph showing the frequency characteristics of the positive electrode;
FIG. 27 is a graph showing the frequency characteristics of the negative electrode;
FIG. 28 is a graph showing the relationship between the frequency and the measurement error when the negative electrode resistance is 5 kΩ;
FIG. 29 is a graph showing the relationship between the frequency and the measurement error when the positive electrode resistance is 10 kΩ;
FIG. 30 is a graph showing the relationship between the frequency and the measurement error when the negative electrode resistance is 25 kΩ;
FIG. 31 is a block diagram showing the structure of a glucose concentration analyzer of the second embodiment of the present invention;
FIG. 32 is a block diagram showing the structure of a glucose concentration analyzer of the third embodiment of the present invention;
FIG. 33 is a flow chart illustrating the measuring operation of the conductance measuring apparatus of the glucose concentration analyzer shown in FIG. 32; and
FIG. 34 is a flow chart illustrating the analyzing operation of the concentration analyzer of the glucose concentration analyzer shown in FIG. 32.

DETAILED DESCRIPTION OF THE EMBODIMENT

The embodiments of the present invention are described hereinafter based on the drawings.

First Embodiment

FIG. 1 is a block diagram showing the structure of a glucose concentration analyzer of the first embodiment of the present invention. The structure of the glucose concentration analyzer 100 of the first embodiment of the present invention is first described below, referring to FIG. 1.

As shown in FIG. 1, the glucose concentration analyzer 100 is an apparatus which extracts tissue fluid that contains the biological component glucose from a living body, and calculates the blood sugar level by analyzing the glucose contained in the extracted tissue fluid. In the glucose concentration analyzer 100, tissue fluid is extracted from an extraction region by applying a direct current to the skin (extraction region) of a subject where micropores have been formed, and the amount of glucose (glucose extraction amount) is obtained from the tissue fluid by a glucose sensor. Since the amount of extracted glucose changes according to the depth of the micropores, the conductance of the extraction region is calculated from electrical information obtained by applying an alternating current to the skin (extraction region) of the subject, and monitoring the condition of the extraction region (depth of micropores and the like) by the conductance of the extraction region. The glucose concentration can therefore be determined more accurately by correcting the glucose extraction amount by the conductance value. Since the concentration of the glucose contained in the tissue fluid is essentially equal to the blood sugar level, the blood sugar level of the subject can be measured without collecting blood from the subject. The structure of the glucose concentration analyzer 100 is discussed in detail below.

The glucose concentration analyzer 100 is provided with a dry electrode 1 formed of Ti and disposed at a region 501 outside of the region 501 on the skin 500 of a subject, a power source 2 for supplying an alternating current and a direct current through current controls, a glucose sensor 3 configured by a semiconductor laser and photodiode, a control and analysis part 4 which controls the power source 2 and the glucose sensor 3, a voltmeter 5, an input part 6 on the glucose concentration analyzer 100 which is configured by buttons or the like for the subject to specify the start of measurement, and a display part 7 for displaying the measurement results and the like. A disposable chip 200, which is replaceable for each measurement when analysis is performed, is mounted on the glucose concentration analyzer 100.

The disposable chip 200 includes a mesh sheet 201 which is permeated with purified water, a sensor member 202 which is painted with a substance that reacts with the glucose contained in the mesh sheet 201 and which is disposed on the top surface of the mesh sheet 201, an electrode 203 formed of Ag/AgCl, a reference electrode 204 formed of Ag/AgCl, and a masking tape 205. When the disposable chip 200 is in the mounted state, the sensor member 202 is disposed at the bottom part of the glucose sensor 3, and the electrode 203 and reference electrode 204 are connected to the power source 2 and the voltmeter 5.

The mesh sheet 201 is configured so as to be capable of holding approximately 3.5 μl of purified water. The mesh sheet 201 is also made of nylon and substantially inelastic so that the thickness does not vary. Specifically, is formed of woven nylon fibers approximately 30 μm in thickness, and has a square net mesh structure approximately 33 μm on a side. The mesh sheet 201 is approximately 10 mm long, approximately 4 mm wide, and approximately 50 μm thick.

The mesh sheet 201 makes contact with the surface of the skin 500 of the subject opposite the extraction region 501. The sensor 202 and electrode 203 are disposed on the top surface of the mesh 201 on the opposite side from the extraction region 501.

On the side facing the mesh sheet 201, the sensor member 202 is painted with an enzyme (glucose oxidase) which acts as a catalyst for glucose, and enzyme (peroxidase) which acts as a catalyst for hydrogen peroxide ($H_2O_2$), and a color-producing agent which reacts with active oxygen. For example, N,N-bis(2-hydroxy-3-sulfopropyl)tolidene potassium salt,3,3',5,5'-tetramethylbenzyldene and the like may be used as the color-producing agent.

The masking tape 205 is provided to prevent direct contact between the reference electrode 204 and the skin 500 of the subject, and to reduce the discomfort of the subject caused by the current flowing to the skin 500. The masking tape 205 is disposed on the bottom surface of the mesh sheet 201 and the reference electrode 204. The masking tape 205 is provided with an orifice 205a which measure approximately 7 $mm^2$. The mesh sheet 201 is configured so as to make contact with the extraction region 501 through these orifices 205a.

When the disposable chip 200 is mounted on the glucose concentration analyzer 100, the power source 2 is configured to apply a current (direct current and alternating current) to the skin 500 of the subject through the electrode 203 and the dry electrode 1. The power source 2 applies a direct current to the skin 500 with the electrode 203 functioning as a negative electrode and the dry electrode 1 functioning as a positive electrode.

The glucose sensor 3 detects the amount of glucose contained in the mesh sheet 201 by measuring the color-producing agent and the like of the sensor member 202. The voltmeter 5 is configured to measure the voltage between the dry electrode 1 and electrode 204 when a current (alternating current) is applied to the skin 500 of the subject through the electrode 203 and dry electrode 1 from the power source 2. The value measured by the voltmeter 5 and the amount of glucose measured by the glucose sensor 3 are input to the control and analysis part 4.

The control and analysis part 4 calculates the impedance of the extraction region 501 using the voltage value received from the voltmeter 5, and calculates the conductance k of the extraction region 501 based on the calculated impedance value. The control and analysis part 4 also calculates the final glucose concentration, that is, essentially the blood sugar level, of the tissue fluid based on the conductance k of the extraction region 501 and the amount of glucose in the mesh sheet 201 which was received from the glucose sensor 3.

FIG. 2 is a perspective view of the micro needles used when forming the minute extraction holes in the skin of the subject in the skin permeability facilitation treatment. FIG. 3 is a cross section view of the skin (extraction region) in which the extraction holes have been formed by the micro needle shown in FIG. 2. The skin permeability facilitation treatment is described below as a form of preprocessing for the measurement of the blood sugar level by the glucose concentration analyzer 100 of the first embodiment referring to FIGS. 2 and 3.

As shown in FIG. 2, a needle roller 600 is configured by an arm 601, and a plurality of rollers 602 supported by the arm 601 so as to be rotatable. A plurality of micro needles 603 are formed with predetermined spacing on the exterior circumferential surface of the roller 602. The needle 603 protrudes (approximately 0.3 mm) to the extend of passing through the dermis, including the corneum of the skin, but does not reach to the subcutaneous tissue when pressed to the skin. As shown in FIG. 3, micro extraction holes 501a are formed through the dermis of the skin to the corium, but do not reach as far as the hypodermis in the preprocessing using the needle roller 600, that is, the process of pressing the needles 603 on the extraction region 501. By forming the extraction holes 501a, tissue fluid containing glucose can be easily extracted from a living body through a plurality of extraction holes 501a in the first embodiment. When the glucose is extracted from a living body using the glucose concentration analyzer 100, the discomfort felt by the subject is therefore reduced. Moreover, a Derma roller manufactured by Top-Rol, Ltd., may be used as the needle roller 600 in the first embodiment.

FIG. 4 is a flow chart illustrating the analyzing operation of the glucose concentration analyzer of the first embodiment shown in FIG. 1. The sequence of the blood sugar level measurement operation using the glucose concentration analyzer 100 of the first embodiment of the present invention is described below with reference to FIGS. 1 through 4.

When the blood sugar level is measured using the glucose concentration analyzer 100 of the first embodiment, micro extraction holes 501a are first formed in the skin 500 of the subject using the needle roller 600 (refer to FIG. 2). Specifically, a plurality of micro extraction holes 501a are formed as shown in FIG. 3 by pressing the needles 603 against the extraction region 501 of the skin. The surface area S of the extraction region 501 is the surface area of the orifice 205a of the masking tape 205 (approximately 7 $mm^2$). Tissue fluid, which includes glucose that permeates the corium of the skin, is gradually exuded through the extraction holes 501a formed in the skin by the preprocessing (skin permeability facilitating treatment) using the needle roller 600.

The subject installs the disposable chip 200 (refer to FIG. 1) on the glucose concentration analyzer 100 after the mesh sheet 201 of the disposable chip 200 is permeated with purified water. At this time, a sensor (not shown in the drawing) in the glucose concentration analyzer 100 determines whether or not a disposable chip 200 is installed in step S1 of FIG. 4. This determination is repeated when a disposable chip 200 is not installed in the apparatus. The routine continues to step S2 when a disposable chip 200 is installed in the apparatus.

The subject subsequently mounts the glucose concentration analyzer 100 on the skin 500 of the subject so that the extraction region 501, in which the extraction holes 501a have been formed, and the negative electrode (mesh sheet 201) are in surface contact. At this time the purified water contained in the mesh sheet 201 penetrates into the extraction holes 501a formed in the skin 500. The tissue fluid within the extraction holes 501a is then dispersed within the purified water held in the mesh sheet 201 when the tissue fluid exuded to the extraction holes 501a of the skin 500 mixes with the purified water from the mesh sheet 201. Tissue fluid from the corium is again exuded to the extraction holes 501a because the osmotic pressure within the extraction holes 501a is lower than the osmotic pressure of the corium of the skin. As a result, the tissue fluid exuded through the extraction holes 501a formed in the skin 500 is dispersed to a degree in the purified water held by the mesh sheet 201 before a current is applied from the power source 2. The subject subsequently issues a measurement start instruction by pressing a button (input part 6) of the glucose concentration analyzer 100.

In the glucose concentration analyzer 100, a determination is also made in step S2 as to whether or not a measurement start instruction has been issued. This determination is repeated when a measurement start instruction has not been issued. When a measurement start instruction has been issued, the power source applies a direct current of approximately 80 μA to the skin 500 for approximately 180 seconds in step S3. The charged ionic components (Na+ and the like) present in the extraction holes 501a therefore actively migrate. Glucose at a level which is detectable by the glucose sensor 3 (refer to FIG. 1) is collected from the living body into the purified water held by the mesh sheet 201 in conjunction with the migration of the ionic components. The glucose collected in the purified water arrives at the sensor member 202 which is disposed on the top surface of the mesh screen 201.

In the glucose concentration analyzer 100, the extraction rate (amount extracted per part time) of glucose extracted to the purified water collected in the mesh sheet 201 is measured in step S4.

Specifically, the glucose that reaches the sensor member 202 reacts with the glucose oxidase which acts as a catalyst, and the resulting product of this reaction, hydrogen peroxide ($H_2O_2$) reacts with the peroxidase which acts as a catalyst. Active oxygen is generated as a result. The color-producing agent painted on the sensor member 202 reacts with the active oxygen and produces a color. The color-producing agent thus produces a color the depth of which accords with the amount of glucose extracted from the living body.

Among the laser light irradiated from the semiconductor laser of the glucose sensor 3, on the other hand, there is a change in the intensity of the light which passes through the sensor member 202 sitting in contact with the mesh sheet 201 containing the purified water because the light intensity of the color produced by the color-producing agent accords with the amount of glucose extracted from the living body.

As a result, a light which has an intensity that accords with the amount of glucose arriving at the sensor member 202 impinges the photodiode of the glucose sensor 3, and a signal is output which corresponds to the intensity of the impinging light. The control and analysis part 4 then calculates the amount of extracted glucose based on the signal output from the photodiode of the glucose sensor 3. The control and analysis part 4 calculates the glucose extraction rate J by dividing the obtained amount of extracted glucose by the time (approximately 180 seconds) during which the power source 2 applied the direct current.

In step S5, the glucose concentration analyzer 100 waits for approximately 10 seconds after the application of the direct current for extracting glucose has terminated. That is, the ions of Na+ and the like disperse and become uniform throughout the mesh sheet 201 during the approximately 10 second waiting time because the ions of Na+ and the like are localized near the active electrode 203 of the mesh sheet 201 immediately after the direct current has been applied.

In the glucose concentration analyzer 100, the power source 2 applies an alternating current (approximately 500 Hz) of approximately ±30 μA to the skin 500 in step S6. This time the measurement value (impedance of the extraction region 501) of the voltmeter 5 is sent to the control and analysis part 4.

In step S7 in the glucose concentration analyzer 100, the conductance of the extraction region 501 is calculated based on the measurement value (impedance of the extraction region 501) of the voltmeter 5 obtained in step S6. That is, the measurement value of the voltmeter 5 approximates the sum of the voltage drop of the extraction region 501 and the voltage drop of the area 502. In the case of a high frequency alternating current (approximately 100 Hz or greater), the measurement value of the voltmeter 5 approximates the voltage drop of the extraction region 501 since the impedance of the area 502 is smaller compared to the impedance of the extraction region 501. The control and analysis part 4 calculates a reciprocal of the impedance of the extraction region 501 as the conductance k of the extraction region 501.

In step S8 the glucose concentration analyzer 100 calculates the glucose concentration (blood sugar level). That is, equation (1) below is established among the blood sugar level C, glucose extraction rate J, surface area S of the extraction region 501, and glucose permeability P of the skin 500 (extraction region 501).

$$J = S \times C \times P \quad \cdot\cdot\cdot (1)$$

The blood sugar level C can therefore be expressed by equation (2) below.

$$C = J / (S \times P) \quad \cdot\cdot\cdot (2)$$

A specific relational equation has been established experimentally for the glucose permeability P and the conductance k of the extraction region 501 calculated in step S6 and is described in US20060029991, and the glucose permeability P can be calculated based on this relational equation.

The glucose extraction rate J is obtained in step S4, and since the surface area S of the extraction region 501 is the surface area of the orifice 205a of the masking tape 205 (approximately 7 $mm^2$), the blood sugar level C can be calculated based on equation (2) above. Furthermore, the calculated blood sugar level can be displayed on the display part 7. The analysis of the blood sugar level is performed as described above by the glucose concentration analyzer 100 of the first embodiment.

Comparative experiments are described below which verify the results using purified water as the medium for holding tissue fluid which contains glucose.

In the comparative experiments, the glucose extraction rate J and conductance k were measured a plurality of times for a plurality of subjects using the glucose concentration analyzer 100 and same measuring sequence as in the first embodiment. Furthermore, the blood sugar level C of the same subject was measured using a different blood sugar-level measuring apparatus (a model manufactured by Nipro Corporation, Ltd.) than the glucose concentration analyzer 100 of the first embodiment. The permeability P was calculated from the blood sugar level C, glucose extraction rate J, and surface area S using equation (1). Data (k, P) of the mutually corresponding glucose permeability P and conductance k were plotted on a coordinate map with the conductance k and permeability P on the horizontal axis and vertical axis, respectively.

The comparative experiments were performed using mesh sheets 201 saturated with media (examples 1~4, and reference example 1) with different concentrations of NaCl concentrations. The concentrations of NaCl of the media used in examples 1~4 and reference example 1 are shown in Table 1 below. Example 1 used purified water as an example of a medium with low conductivity used as the medium in the first embodiment, and reference example 1 used a conventional medium of physiological saline solution. Examples 2~4 used examples of other media with low conductivity.

Table 1

| | NaCl Concentration | Conductance | Resistivity |
|---|---|---|---|
| Example 1 | 0 | 0 | 18300 |
| Example 2 | 0.5 | 59.72 | 16.745 |
| Example 3 | 1 | 119.44 | 8.372 |
| Example 4 | 5 | 597.2 | 1.674 |
| Reference Ex. 1 | 154 | 18393.76 | 0.054 |
| * M=mol/1 | | | |

As shown in Table 1, example 1, which used a concentration of NaCl of 0 mM (purified water), had a conductivity of 0 (μS (seimens)/cm) and resistivity of 18300 (KΩ•cm). Example 2, which used a concentration of NaCl of 0.5 mM, had a conductivity of 59.72 μS/cm) and resistivity of 16.745 (KΩ•cm).

6 Example 3,which used a concentration of NaCl of 1 mM, had a conductivity of 119.44 (μS/cm) and resistivity of 8.372 (KΩ•cm). Example 4, which used a concentration of NaCl of 5 mM, had a conductivity of 597.2 (μS/cm) and resistivity

of 1.674 (KΩ•cm). Reference example 1,which used a concentration of NaCl of 154 mM (physiological saline solution), had a conductivity of 18393.76 (μS/cm) and resistivity of 0.054 (KΩ•cm).

The results of the experiments performed using the examples 1~4 and reference example 1 are shown in FIGS. 5 through 9. As shown in FIGS. 5 through 9, it can be understood that the conductance k and the permeability P have a proportional relationship (correlation). The straight line in FIGS. 5 through 9 represents the line of minimum difference between each point (k, P) (that is, a regression line) determined by the method of least squares. In FIG. 5, for example P=112.84 k + 2.6793. R2 in FIGS. 5 through 9 is a so-called correlation coefficient, and represents the strength of the correlation between the conductance k and the permeability P. The correlation coefficient R2 indicates a high correlation of a value approaching [1] when the conductance k and permeability P have a positive proportional correlation of values [0] to [1]. When all the plurality of points (k, P) are on the same straight line, the correlation coefficient is [1]. As shown in FIG. 5, the correlation coefficient R2=0.8554 in example 1, and it is understood that the conductance k and permeability P have a high correlation. as shown in FIG. 9, the correlation coefficient R2=0.1705 in the reference example 1, and it is understood that the conductance k and permeability P have a low correlation. In examples 2 through 4 shown in FIGS. 6 through 8, the correlation coefficients R2=0.5809, R2=0.6939, R2=3301, respectively, and it is understood that the conductance k and permeability P have high correlations.

Measurement errors were then determined in the experimental results of examples 1~4 and reference example 1. The measurement errors of example 1 are described in detail below with reference to FIG. 5. The divergence rate of the estimated permeability of the glucose permeability (actual permeability) is calculated at each point by dividing the glucose permeability (actual permeability) at each point (k, P) by the glucose permeability (estimated permeability) on the regression line in FIG. 5 (k: 112.84 k + 2.6793), and multiplying by 100. That is, the divergence rate can be expressed by equation (3) below.

$$\text{Divergence rate} = (\text{actual permeability}/\text{estimated permeability})\times 100 =$$

$$(P/(112.84k+2.6793))\times 100 \ \ldots(3)$$

Thereafter, the standard deviation and average value are calculated from the divergence rate at each point, and the measurement error is calculated fusing equation (4) below.

$$\text{Measurement error} + (\text{standard deviation}/\text{average value})\times 100 \ldots(4)$$

The measurement error was calculated in the manner for examples 1 through 4 and the reference example 1. The calculation results are shown in Table 2 below and in FIG. 10.

Table 2

| | NaCl Concentration (mM)* | Measurement Error (%) |
|---|---|---|
| Example 1 | 0 | 9.34057 |
| Example 2 | 0.5 | 15.2159 |
| Example 3 | 1 | 18.143 |
| Example 4 | 5 | 18.8571 |
| Reference Ex. 1 | 154 | 24.0272 |
| * M=mol/l | | |

As shown in Table 2, the measurement error of example 1 was 9.34057, and the measurement error of example 2 was 15. 2159. The measurement error of example 3 was 18.143, and the measurement error of example 4 was 18.8571. Since the measurement error of reference example 1 was 24.0272, the measurement errors of examples 1 through 4 are clearly improvements over the measurement error of reference example 1. Thus, the measurement error becomes smaller as the concentration of NaCl becomes lower, as shown in Table 2 and FIG. 10. The measurement error is substantially lower in example 1 (purified water) compared to reference example 1 (physiological saline solution).

The mechanism by which the above experimental results were obtained is considered below. FIGS. 11 and 12 are conceptual views respectively illustrating the behavior of ions in the medium when purified water (example 1) and physiological saline solution (reference example 1) were used as the medium for holding tissue fluid.

The tissue fluid extracted from the skin (extraction region) of the subject contains sodium ion (Na+) as well as glucose. When a direct current is applied to extract the glucose, chloride ions (Cl-) and sodium ions (Na+) are forcibly diffused from the electrodes (Ag/AgCl) and the skin (extraction region) by the electric field which forms. Tissue fluid that contains glucose is extracted into the medium because the glucose also migrates in conjunction with the forcible diffusion of the sodium ions. Thus, the behavior of the sodium ions is thought to be closely associated with the mechanism by which glucose is extracted.

A current flows by means of the charges carried by the sodium ions and chloride ions. The percentage of the current flowing by the chloride ions as a medium at this time can be designated the transport rate tCl of the chloride ions, and the percentage of the current flowing by the sodium ions as a medium at this time can be designated the transport rate tNa of the sodium ions (tCl+tNa=1).

As shown in FIG. 11, when purified water is used as the medium (example 1), sodium ions (Na+) are diffused from the skin to render the medium electrically neutral when chloride ions (Cl-) are diffused to the medium from the electrode since purified water does not contain any chloride ions (Cl-) and sodium ions (Na+). Since sodium ions are unilaterally diffused from the skin, the sodium ion transport rate tNa near the skin (extraction region) can be considered to be stabilized at a high value (tNa=1) which is unaffected by the condition of the skin (micropore depth and size). In example 1, there is though to be good correspondence between the glucose permeability P and the conductance k which reflects the condition of the skin since the behavior of the sodium ions, which carry out the extraction of the glucose as described above, is stable and unaffected by the condition of the skin.

When physiological saline solution is used as the medium (reference example 1), the medium contains large amounts of chloride ions (Cl-) and sodium ions (Na+), as shown in FIG. 12. Therefore, two cases can be considered, one being when the chloride ions (Cl-) are diffused from the electrode into the medium, and the other when the chloride ions (Cl-) in the medium enter the skin as the sodium ions (Na+) are diffused from the skin in order to maintain electrical neutrality in the medium. When physiological saline solution is used as the medium, a dispersion is therefore believed to occur and the transport rate tNa of the sodium ions near the skin (extraction region) is a low value (tNa=0.7) depending on the condition of the skin (size and depth of the micropores). There is though to be a poor correlation between the glucose permeability P and the conductance k which reflects the condition of the skin in reference example 1, due to the deviation in the behavior of the sodium ions which carry out the extraction of the glucose because of the condition of the skin.

In the first embodiment described above, excellent correlation can be attained between the permeability of glucose through the extraction region 501 and the conductance of the extraction region 501 by using purified water as the medium for holding tissue fluid that contains glucose, compared to using an electrically conductive medium such as physiological saline or the like which has low resistivity of approximately 0.054. The permeability of glucose through the extraction region can therefore be estimated with greater accuracy by measuring the conductance of the extraction region 501.

In the first embodiment described above, tissue fluid can be easily extracted through the extraction region 501 into the purified water within the mesh sheet 201 by forming micropores (extraction holes 501a) in the extraction region 501 as a preprocess.

In the first embodiment described above, the conductance of the extraction region 501 can be measured in a relatively short time using a relatively simple construction by applying an alternating current only between the electrode 203 and the dry electrode 1.

In the first embodiment described above uses a mesh sheet 201 of relatively small capacity of approximately 3.5 μl, and which has a relatively small thickness of approximately 50 μ, width of approximately 4 mm, and length of approximately 10 mm, as the medium for holding the purified water, so that ions can be uniformly diffused in the mesh sheet 201 immediately after extraction of the tissue fluid localized near the electrode 203 where the ions are present by implementing a relatively short waiting time compared to using a relatively large capacity of medium. thus, the conductance can be rapidly obtained by starting the current flow to the electrode 203 and the dry electrode 1.

Second Embodiment

FIG. 13 is a block diagram showing the structure of a glucose concentration analyzer of the second embodiment of the present invention. The second embodiment differs from the first embodiment, and is described by way of example in which the conductance measuring apparatus which calculates the conductance is separate from the concentration analyzer which calculates the glucose concentration. The structure of the glucose concentration analyzer 300 of the second embodiment of the present invention is first described below referring to FIG. 13.

The glucose concentration analyzer 300 of the second embodiment is configured by a conductance measuring apparatus 300a which is mounted on the skin 500 of a subject to calculate the conductance of the extraction region 501a and extract glucose to the mesh sheet 201, and a concentration analyzer 300b which calculates the glucose extraction rate from the mesh sheet 201 that contains glucose, and calculates the concentration of the glucose (blood sugar level) from the glucose extraction rate and the conductance calculated by the conductance measuring apparatus 300a.

The structure of the conductance measuring apparatus 300a is identical to the structure of the glucose concentration analyzer 100 of the first embodiment with the exception that the glucose sensor 3 is omitted, and the description is therefore abbreviated. When glucose is extracted in the conductance measuring apparatus 300a, a disposable chip 200a is mounted at the extraction region 501 similar to the first embodiment. The disposable chip 200a of the second embodiment has a configuration in which the sensor member 202 is omitted from the disposable chip 200 of the first embodiment.

The concentration analyzer 300b is provided with a receiving part 301 for receiving the mesh sheet 201 which contains the glucose obtained in the conductance measuring apparatus 300a, a glucose sensor 302 for measuring the amount of glucose extracted from the mesh part 201, a control and analysis part 303 for calculating the glucose extraction rate from the amount of extracted glucose obtained by the glucose sensor 302, an input part 304 configured by buttons or the like for starting the analysis by the concentration analyzer 300b, and a display part 305 for displaying analysis results and the like. A sensor member 306, which has a configuration identical to that of the sensor member 202 of the first embodiment, is disposed on the glucose sensor 302 side of the receiving part 301 when performing the analysis of glucose concentration in the concentration analyzer 300b.

The control and analysis part 303 is connected by wire or wirelessly to the control and analysis part 4 of the conductance measuring apparatus 300a, and is capable of receiving the conductance value and the current application time of the direct current applied to extract the tissue fluid from the control and analysis part 4 of the conductance measuring apparatus 300a.

FIGS. 14 and 15 are flow charts illustrating the operation of the concentration analyzer and the conductance measuring apparatus of the glucose concentration analyzer of the second embodiment of the present invention. The analysis operation performed by the glucose concentration analyzer 300 of the second embodiment of the present invention is described below with reference to FIGS. 2~4 and FIGS. 13~15.

When the blood sugar level is measured using the glucose concentration analyzer 300 of the second embodiment, extraction holes 501a (refer to FIG. 3) are first formed in the skin 500 of the subject using the needle roller 600 (refer to FIG. 2).

The subject installs the disposable chip 200a in the conductance measuring apparatus 300a of the glucose concentration analyzer 300. At this time, the conductance measuring apparatus 300a determines whether or not a disposable chip 200a is installed in the apparatus in step S1 of FIG. 14. This determination is repeated when a disposable chip 200a is not installed in the apparatus. The routine continues to step S12 when a disposable chip 200a is installed in the apparatus.

Thereafter, the conductance measuring apparatus 300a is mounted on the skin 500 of the subject so that the extraction region 501 in which the extraction holes 501a have been formed, and the negative electrode (mesh sheet 201 of the disposable chip 200a) are in contact. The subject subsequently issues a measurement start instruction by pressing the button (input part 6) of the conductance measuring apparatus 300a.

The conductance measuring apparatus 300a also determines whether or not a measurement start instruction has been issued in step S12. This determination is repeated when a measurement start instruction has not been issued. When the measurement start instruction has been issued, a direct current is applied to the skin 500 in step S13 in the same manner as step S3 of the first embodiment (refer to FIG. 4). Thus, glucose is extracted to the mesh sheet 201.

The conductance measuring apparatus 300a waits approximately 10 seconds in step S14 immediately after the application of the direct current for extracting glucose has been terminated similar to step S5 of the first embodiment (refer to FIG. 4). Thus, the ions of Na+ and the like attain a state of homogeneity in the purified water within the mesh sheet 201.

In the conductance measuring apparatus 300a, an alternating current is subsequently applied to the skin in step S15 and the conductance of the extraction region 501 is calculated in step S16 similar to steps S6 and S7 of the first embodiment (refer to FIG. 4).

In step S17, the conductance measuring apparatus 300a sends the conductance of the extraction region 501 calculated in step S16 from the control and analysis part 4 of the conductance measuring apparatus 300a to the control and analysis part 303 of the concentration analyzer 300b. The operation of the conductance measuring apparatus 300a thus ends.

The subject then removes the disposable chip 200a from the conductance measuring apparatus 300a, and installs the disposable chip 200a in the concentration analyzer 300b.

The concentration analyzer 300b first determines whether or not a sensor member 306 is installed in step S21. This determination is repeated when the sensor member 306 has not been installed. When a sensor member 306 has been installed the routine continues to step S22.

In step S22 the concentration analyzer 300b determines whether or not the disposable chip 200a has been installed. This determination is repeated when a disposable chip 200a is not installed. The routine continues to step S23 when a disposable chip 200a is installed.

The subject issues a measurement start instruction by operating the input part 304, which is configured by a button or the like, after the disposable chip 200a has been installed in the concentration analyzer 300b. In the glucose concentration analyzer 300a, a determination is also made in step S23 as to whether or not a measurement start instruction has been issued. This determination is repeated when a measurement start instruction has not been issued. When a measurement start instruction has been issued, the control and analysis part 303 calculates the amount of extracted glucose in step S24 based on the signal output from the photodiode to the glucose sensor 302 similar to step S4 of the first embodiment (refer to FIG. 4).

In step S25, a determination is then made in the concentration analyzer 300b as to whether or not information such as the conductance of the extraction region 501 has been received from the conductance measuring apparatus 300a. This determination is repeated when information has not been received. When information has been received the routine continues to step S26. The information of conductance and the like includes the application time of the direct current applied to extract the glucose in step S13 (refer to FIG. 14).

In step S26, the glucose concentration (blood sugar level) is calculated in the concentration analyzer 300b. That is, the control and analysis part 303 calculates the glucose extraction rate J by dividing the amount of extracted glucose obtained in step S24 by the application time of the direct current received in step S25. The control and analysis part 303 then calculates the glucose concentration (blood sugar level) of the tissue fluid based on the conductance k of the extraction region 501 and the glucose extraction rate.

Similar to the first embodiment, the second embodiment provides excellent correlation between the conductance and glucose permeability by using purified water as a medium for holding glucose. The blood sugar level can therefore be calculated with greater accuracy.

In the second embodiment described above, it is possible to prevent substances such as glucose oxidase, peroxidase, and color-producing agent contained in the sensor member 306, which are required to measure the amount of extracted glucose, from coming into contact with the skin of the subject by separating the conductance measuring apparatus 300a which measures conductance and extracts glucose, and the concentration analyzer 300b which is provided with the glucose sensor 302 that obtains the amount of extracted glucose while mounted on the skin of the subject. The safety of the subject s therefore increased.

The other effects of the second embodiment are identical to the effects of the first embodiment.

Third Embodiment

FIG. 16 is a block diagram showing the structure of a glucose concentration analyzer of the third embodiment of the present invention. The third embodiment differs from the first embodiment and second embodiment, and is described by way of example of measuring the conductance by a direct current. The structure of the glucose concentration analyzer 400 of the third embodiment of the present invention is first described below referring to FIG. 16.

As shown in FIG. 16, the glucose concentration analyzer 400 of the third embodiment is provided with a chamber 401 which is disposed on the extraction region 501 of the skin 500 of the subject, a syringe 402 for supplying purified water to the chamber 401, a glucose sensor 403 provided in the chamber 401, an electrode 404 disposed in the chamber 401, and electrodes 405 and 406 which are respectively disposed on the regions 502 and 503 which are outside the extraction region 501 of the skin 500. The glucose concentration analyzer 400 is further provided with a power source 407 for applying a direct current to the skin 500 through the electrodes 404, 405 and 406, a switching circuit 408 for switching the current flow pattern, a voltmeter 409 for measuring the output of the power source 407, a control and analysis part 410 for controlling the glucose sensor 403, power source 407, switching circuit 408 and the like, an input part 411, and a display part 412.

The chamber 401 is capable of accommodating approximately 80 $\mu$l of purified water. The purified water supplied to the chamber 401 has the function of holding the glucose which is extracted from the extraction region 501. The glucose sensor 403 is configured by the glucose sensor 3 and the sensor member 202 of the first embodiment. The amount of glucose holded in the purified water of the chamber 401 is measured by the glucose sensor 403. The amount of glucose is sent to the control and analysis part 410.

The switching circuit 408 is capable of switching among a first current flow pattern in which a current flows through the electrodes 404 and 405 and the skin 500, a second current flow pattern in which a current flows through the electrodes 404 and 406 and the skin 500, and a third current flow pattern in which a current flows through the electrodes 405 and 406 and the skin 500.

The voltmeter 409 is provided to measure the voltage value in the current flow patterns which are respectively switched by the switching circuit 408. This current value is sent to the control and analysis part 410. The control and analysis part 410 is configured to calculate the resistance Ra of the extraction region 501 based on the voltage value, and calculate the conductance of the extraction region 501 based on the calculated resistance Ra.

Since the input part 411 and the display part 412 are identical to the input part 6 and display part 7 of the first embodiment, further description is omitted.

FIGS. 17 through 19 are equivalence circuit diagrams respectively illustrating the first current flow pattern, second current flow pattern, and third current flow pattern of the glucose concentration analyzer shown in FIG. 16. The conductance measurement principle employed by the glucose concentration analyzer 400 of the third embodiment is described below with reference to FIGS. 17 through 19.

In the first current flow pattern as shown in FIG. 17, the output value of the voltmeter 409 is a resistance value Rab between the extraction region 501 and the region 502. The resistance value Rab between the extraction region 5012 and the region 502 can be expressed as the sum of the resistance value Ta of the extraction region 501, the resistance value Rb of the region 502, and the resistance value Rd of the tissue on the interior side of the skin 500 (extraction region 501, region 502, and region 503). The resistance value Rab can be expressed by equation (5) below.

$$Rab = Ra + Rb + Rd \quad \cdots (5)$$

Since the resistance of the tissue on the interior side of the skin 500 is sufficiently lower than the resistance of the skin 500, the relationships Rd<<Ra+Rb and 2Rd<<Ra+Rb are established. Equation (5) therefore approximates equation (6) below.

$$Rab = Ra + Rb \quad \cdots (6)$$

In the second current flow pattern as shown in FIG. 18, the output value of the voltmeter 409 is a resistance value Rac between the extraction region 501 and the region 503. The resistance value Rac can be approximated by equation (7) below similar to the case of the first current flow pattern.

$$Rac = Ra + Rc + 2Rd = Ra + Rc \quad \cdots (7)$$

In the third current flow pattern as shown in FIG. 19, the output value of the voltmeter 409 is a resistance value Rbc between the region 502 and the region 503. The resistance value Rbc can be approximated by equation (8) below similar to the case of the first current flow pattern.

$$Rbc = Rb + Rc + Rd = Rb + Rc \quad \cdots (8)$$

From equations (6), (7), and (8), the resistance Ra of the extraction region 501 can be expressed by equation (9) below.

$$Ra = (Rab + Rac - Rbc) / 2 \quad \cdots (9)$$

Thus, the resistance Ra of the extraction region 501 can be calculated from the output values of the voltmeter in the respective first current flow pattern, second current flow patter, and third current flow pattern. The conductance k of the extraction region 501 is a reciprocal of the resistance value Ra of the extraction region 501, and can be expressed by equation (10) below.

$$k = 1 / Ra \quad \cdots (10)$$

The conductance calculation is thus performed in the glucose concentration analyzer 400 of the third embodiment.

FIG. 20 is a flow chart illustrating the analysis operation of the glucose concentration analyzer 400 of the third embodiment. The analysis operation of the glucose concentration analyzer 400 is described below with reference to FIGS. 3, 4, and 16 through 20.

When a blood sugar level is measured using the glucose concentration analyzer 400 of the third embodiment, the subject first forms extraction holes 501a (refer to FIG. 3) in preprocessing of the extraction region 501 similar to the first embodiment.

The subject thereafter installs the glucose concentration analyzer 400 on the skin 500 of the subject so that the chamber 401 is positioned on the extraction region 501 in which the extraction holes 501a have been formed. Then approximately 80 μl of purified water is supplied into the chamber 401 by the syringe 402. The subject subsequently starts the measurement by pressing a button (input part 411) of the glucose concentration analyzer 400.

In the glucose concentration analyzer 400, a determination is made in step S31 of FIG. 20 as to whether or not a measurement start instruction has been issued. This determination is repeated when a measurement start instruction has not been issued. When a measurement start instruction has been issued, the power source 407 applies a direct (DC) current of approximately 80 μA for approximately 180 seconds to the skin 500 using the electrode 404 as a negative electrode and the electrode 405 as the positive electrode in step S32. Therefore, glucose is collected in the purified water held within the chamber 401 through the extraction region 501. The glucose collected in the purified water reaches a sensor member (not shown in the drawing) of the glucose sensor 403 which is disposed on the top surface of the chamber 401.

In step S33, the extraction rate J (amount extracted per part time) of the glucose extracted to the chamber 401 is measured by the glucose sensor 403 in the glucose concentration analyzer 400 similar to step S4 (refer to FIG. 4) of the first embodiment.

In step S34, the glucose concentration analyzer 400 waits for approximately six minutes seconds after the application of the direct current for extracting glucose has terminated. That is, the waiting time is longer in the third embodiment than the waiting time (approximately 10 seconds) in the first embodiment due to the relatively large amount of purified water within the chamber 401. The ions therefore attain a uniform condition within the chamber 401.

In step S35 in the glucose concentration analyzer 400, the switching circuit 408 is switched to the first current flow pattern (refer to FIG. 17), and the power source 2 applies a direct current of approximately 80 μA to the skin 500. The measurement value of the voltmeter 409 (resistance value Rab) is sent to the control and analysis part 410 at this time.

In step S36 in the glucose concentration analyzer 400, the switching circuit 408 is switched to the second current flow pattern (refer to FIG. 18), and the power source 2 applies a direct current of approximately 80 μA to the skin 500. The measurement value of the voltmeter 409 (resistance value Rac) is sent to the control and analysis part 410 at this time.

In step S37 in the glucose concentration analyzer 400, the switching circuit 408 is switched to the third current flow pattern (refer to FIG. 19), and the power source 2 applies a direct current of approximately 80 (A to the skin 500. The measurement value of the voltmeter 409 (resistance value Rbc) is sent to the control and analysis part 410 at this time.

In step S38 in the glucose concentration analyzer 400, the control and analysis part 410 calculates the resistance Ra of the extraction region 501 using equations (9) and (10), and calculates the conductance k which is a reciprocal of the resistance Ra. The glucose permeability P relative to the skin (extraction region 501) is then calculated based on the conductance k.

In step S39 in the glucose concentration analyzer 400, the control and analysis part 410 calculates the glucose concentration (blood sugar level). That is, The glucose concentration C is calculated based on the glucose extraction rate J obtained in step S33, the glucose permeability P and surface area S of the extraction region 501 obtained in step S38, and equation (2). The calculated glucose concentration (blood sugar level) is thereafter displayed on the display part 412. The glucose concentration is calculated as described above in the third embodiment.

Similar to the first embodiment, the third embodiment provides excellent correlation between the conductance and glucose permeability by using purified water as a medium for holding glucose. The blood sugar level can therefore be calculated with greater accuracy.

Conductance can be stably measured in the third embodiment by measuring the conductance of the extraction region 501 by applying a direct current in three current flow patterns using the three electrodes 404, 405, and 406, compared to measuring conductance using an alternating current as in the first and second embodiments.

## Fourth Embodiment

FIG. 23 is a block diagram showing the structure of a glucose concentration analyzer of the fourth embodiment of the present invention. The structure of the glucose concentration analyzer 1000 of fourth embodiment of the present invention is described below with reference to FIG. 23.

As shown in FIG. 23, the glucose concentration analyzer 1000 is an apparatus which extracts tissue fluid that contains the biological component glucose from a living body, and calculates the blood sugar level by analyzing the glucose contained in the extracted tissue fluid. In the glucose concentration analyzer 1000, tissue fluid is extracted from an extraction region by applying a direct current to the skin (extraction region) of a subject where micropores have been formed, and the amount of glucose (glucose extraction amount) is obtained from the tissue fluid by a glucose sensor. Since the amount of extracted glucose changes according to the depth of the micropores, the conductance of the extraction region is calculated from electrical information obtained by applying an alternating current to the skin (extraction region) of the subject, and monitoring the condition of the extraction region (depth of micropores and the like) by the conductance of the extraction region. The glucose concentration can therefore be determined more accurately by correcting the glucose extraction amount by the conductance value. Since the concentration of the glucose contained in the tissue fluid is essentially equal to the blood sugar level, the blood sugar level of the subject can be measured without collecting blood from the subject. This is described in detail below.

The glucose concentration analyzer 1000 is provided with an electrode 1001 which is formed of Ag/AgCl and is positioned on the extraction region 501 of the skin 500 of the subject, a dry electrode 1002 which is formed of Ti and is positioned on region 502 that is outside of the extraction region 501, and a power source 1003 which applies a controlled direct current and alternating current to the skin 500 of the subject through the electrode 1001 and dry electrode 1002. The power source 1003 applies a direct current to the skin 500 with the electrode 1003 functioning as a negative electrode and the dry electrode 1002 functioning as a positive electrode. Furthermore, disposed between the electrode 1001 and the extraction region 501 is a disposable chip 1006, which includes the integratedly configured mesh sheet 1004 that is saturated with purified water and a sensor member 1005 that is painted with substances which react with the glucose contained in the mesh sheet 1004 and is disposed on the top surface of the mesh sheet 1004. Above the disposable chip 1006 is positioned a glucose sensor 1007 that is configured by a semiconductor laser and photodiode. The amount of glucose contained in the mesh sheet 1004 is detected when the glucose sensor 1007 measured the color intensity of the sensor member 1005. The power source 1003 and the glucose sensor 1007 are controlled by a control and analysis part 1008. A reference electrode 1009, which is formed of Ag/AgCl and disposed in substantial contact with the extraction region 501, is configured to measure the voltage between the dry electrode 1002 and the reference electrode 1009 using a voltmeter 1010. The value measured by the voltmeter 1010 and the amount of glucose measured by the glucose sensor 1007 are input to the control and analyzing part 1008. The glucose concentration analyzer 1000 is also provided with an input part 1011 which is configured by a button or the like to allow a subject to start a measurement by the glucose concentration analyzer 1000, and a display part 1012 for displaying the measurement result and the like.

On the side facing the mesh sheet 1004, the sensor member 1005 is painted with an enzyme (glucose oxidase) which acts as a catalyst for glucose, and an enzyme (peroxidase) which acts as a catalyst for hydrogen peroxide ($H_2O_2$), and a color-producing agent which reacts with active oxygen. For example, N,N-bis(2-hydroxy-3-sulfopropyl)tolidene potassium salt,3,3',5,5'-tetramethylbenzyldene and the like may be used as the color-producing agent.

The control and analyzing part 1008 calculates the impedance of the extraction region 501 using the voltage value received from the voltmeter 1010, and calculates the conductance k of the extraction region 501 based on the calculated impedance value. The control and analyzing part 1008 also calculates the final glucose concentration, that is, essentially the blood sugar level, of the tissue fluid based on the conductance k of the extraction region 501 and the amount of glucose in the mesh sheet 1004 which was received from the glucose sensor 1007.

In the fourth embodiment, a Derma roller, manufactured by Top-Rol, Ltd., may be used as the needle roller 600 shown in FIG. 2.

FIG. 24 is a flow chart illustrating the analyzing operation of the glucose concentration analyzer of the fourth embodiment shown in FIG. 23. The sequence of the blood sugar level measurement operation using the glucose concentration analyzer 1000 of the fourth embodiment of the present invention is described below with reference to FIGS. 2, 3, 23, and 24.

When the blood sugar level is measured using the glucose concentration analyzer 1000 of the fourth embodiment, micro extraction holes 501a are first formed in the skin 500 of the subject using the needle roller 600 (refer to FIG. 2). Specifically, a plurality of micro extraction holes 501a are formed as shown in FIG. 3 by pressing the needles 603 against the extraction region 501 of the skin. Tissue fluid, which includes glucose that permeates the corium of the skin, is gradually exuded through the extraction holes 501a formed in the skin by the preprocessing (skin permeability facilitating treatment) using the needle roller 600.

The subject installs the disposable chip 1006 (refer to FIG. 23), which includes the integratedly configured sensor member 1005 and mesh sheet 1004, in the glucose concentration analyzer 1000. At this time, a sensor (not shown in the drawing) in the glucose concentration analyzer 1000 determines whether or not a disposable chip 1006 is installed in step S41 of FIG. 24. This determination is repeated when a disposable chip 1006 is not installed in the apparatus. The routine continues to step S42 when a disposable chip 1006 is installed in the apparatus.

The glucose concentration analyzer 100 is thereafter mounted on the skin 500 of the subject so that the negative electrode (mesh sheet 1004) is in contact with the extraction region 501 in which the extraction holes 501a have been formed. At this time the purified water contained in the mesh sheet 1004 penetrates into the extraction holes 501a formed in the skin 500. The tissue fluid within the extraction holes 501a is then dispersed within the purified water held in the mesh sheet 1004 when the tissue fluid exuded to the extraction holes 501a of the skin 500 mixes with the purified water from the mesh sheet 1004. Tissue fluid from the corium is again exuded to the extraction holes 501a because the osmotic pressure within the extraction holes 501a is lower than the osmotic pressure of the corium of the skin. As a result, the tissue fluid exuded through the extraction holes 501a formed in the skin 500 is dispersed to a degree in the purified water held by the mesh sheet 1004 before a current is applied from the power source 1003. The subject subsequently starts the measurement by pressing a button (input part 1011) of the glucose concentration analyzer 1000.

In the glucose concentration analyzer 1000, a determination is also made in step S42 as to whether or not a measurement start instruction has been issued. This determination is repeated when a measurement start instruction has not been issued. When a measurement start instruction has been issued, the power source 1003 applies a direct current of approximately 80 μA to the skin 500 for approximately 180 seconds in step S43. The charged ionic components present in the extraction holes 501a therefore actively migrate. Glucose at a level which is detectable by the glucose sensor 1007 (refer to FIG. 23) is collected from the living body into the purified water held by the mesh sheet 1004 in conjunction with the migration of the ionic components. The glucose collected in the purified water arrives at the sensor member 1005 which is disposed on the top surface of the mesh screen 1004.

In the glucose concentration analyzer 1000, the extraction rate (amount extracted per part time) of glucose extracted to the purified water collected in the mesh sheet 1004 is measured in step S44.

Specifically, the glucose that reaches the sensor member 1005 reacts with the glucose oxidase which acts as a catalyst, and the resulting product of this reaction, hydrogen peroxide ($H_2O_2$), reacts with the peroxidase which acts as a catalyst. Active oxygen is generated as a result. The color-producing agent painted on the sensor member 1005 reacts with the active oxygen and produces a color. The color-producing agent thus produces a color the depth of which accords with the amount of glucose extracted from the living body.

Among the laser light irradiated from the semiconductor laser of the glucose sensor 1007, on the other hand, there is a change in the intensity of the light which passes through the sensor member 1005 sitting in contact with the mesh sheet 1004 containing the purified water because the light intensity of the color produced by the color-producing agent accords with the amount of glucose extracted from the living body.

As a result, a light, which has an intensity that accords with the amount of glucose arriving at the sensor member 1005, impinges the photodiode of the glucose sensor 1007 and a signal is output which corresponds to the intensity of the impinging light. The control and analyzing part 1008 then calculates the amount of extracted glucose based on the signal output from the photodiode of the glucose sensor 1007. The control and analyzing part 1008 calculates the glucose extraction rate J by dividing the obtained amount of extracted glucose by the time (approximately 90 seconds) during which the power source 1003 applied the direct current.

In the glucose concentration analyzer 1000, the power source 1003 applies an alternating current (approximately 500 Hz) of approximately ±30 μA to the skin 500 in step S45. The measurement value of the voltmeter 1010 (impedance) is sent to the control and analysis part 1008 at this time.

In step S46 in the glucose concentration analyzer 1000, the conductance of the extraction region 501 is calculated based on the measurement value of the voltmeter 1010 obtained in step S45. That is, the measurement value of the voltmeter 1010 approximates the sum of the voltage drop of the extraction region 501 and the voltage drop of the area 502 as described later. In the case of a high frequency alternating current (approximately 100 Hz or greater), the measurement value of the voltmeter 1010 approximates the voltage drop of the extraction region 501 since the impedance of the area 502 is smaller compared to the impedance of the extraction region 501. The control and analyzing part 1008 calculates a reciprocal of the impedance of the extraction region 501 as the conductance k of the extraction region 501.

In step S47 the glucose concentration analyzer 1000 then calculates the glucose concentration (blood-sugar level). That is, equation (1) below is established among the blood sugar level C, glucose extraction rate J, surface area S of the extraction region 501, and glucose permeability P of the skin 500.

$$J = S \times C \times P \quad \cdots (1)$$

The blood sugar level C can therefore be expressed by equation (2) below.

$$C = J / (S \times P) \quad \cdots (2)$$

A specific relational equation has been established experimentally for the glucose permeability P and the conductance k of the extraction region 501 calculated in step S6 and is described in US20060029991, and the glucose permeability P can be calculated based on this relational equation. The glucose extraction rate J was obtained in step S44, and the blood sugar level C can be calculated based on equation (2) since the surface area S of the extraction region 501 is known.

In the fourth embodiment described above, the impedance of the extraction region 501 can be obtained using two electrodes by providing the power source 1003 which applies an alternating current of approximately 500 Hz to the skin 500 of a subject through the electrode 1001 and the dry electrode 1002, and providing the voltmeter 1010 which obtains the impedance of the extraction region 501 by applying the alternating current from the power source 1003 to the skin 500. The conductance of the extraction region 501 of the subject can be calculated based on the impedance of the extraction region 501. Since the impedance of the extraction region 501 can be calculated by applying an alternating current to two electrodes, it is unnecessary to apply a direct current to three electrodes to form three current flow patterns, and thus the conductance of the extracting region can be calculated in a short time. Furthermore, since the conductance can be calculated by applying an alternating current for a short time, electric power consumption can be reduced compared to when a direct current is applied for a long time. The structure of the apparatus can be simplified since the conductance can be calculated using two electrodes, and it is unnecessary to provide a switching circuit for switching the current flow patterns.

In the fourth embodiment, tissue fluid which contains glucose can be easily extracted from the extraction region 501 by forming micro extraction holes 501a in the extraction region 501.

In the fourth embodiment, the voltage between the dry electrode 1002 and the extraction region 501 can be measured without including values which cause error such as the resistance of the mesh sheet 1004 and the resistance of the electrode 1001 and the like, by measuring the voltage between the dry electrode 1002 and the extraction region 501 using the reference electrode. The impedance of the extraction region 501 can be obtained using this voltage.

In the fourth embodiment, the glucose concentration in the tissue fluid can be calculated based on the conductance of the extraction region 501 and the amount of extracted glucose obtained by the glucose sensor 1007 by providing the glucose sensor 1007 which obtains the amount of glucose extracted from the tissue fluid, and the control and analysis part 1008 which calculates the glucose concentration in the tissue fluid based on the glucose extraction amount and the conductance of the extraction region 501. The blood sugar level of a subject can be obtained by the glucose concentration analyzer 1000 of the fourth embodiment since the glucose concentration in the tissue fluid is approximately equal to the blood sugar level.

FIG. 25 is a circuit diagram equivalent to the glucose concentration analyzer of the fourth embodiment shown in FIG. 23. The principle by which the conductance of the extraction region 501 is measured by the glucose concentration analyzer 1000 of the fourth embodiment of the present invention is described below.

In FIG. 25, Rele, Rsol, and Rele-ref respectively refer to the resistance of the electrode 1001, the resistance of the mesh sheet 1004, and the resistance of the reference electrode 1009. The dry electrode 1002 and the region 502m which is outside the extraction region 501 of the skin 500 of the subject, are replaced as the parallel circuits of Cintact (capacitance component) and Rintact (resistance component). similarly, the extraction region 501 of the skin of the subject is replaced as the parallel circuits Cpore (capacitance component) and Rpore (resistance component). The total impedance of Cintact (capacitance component) and Rintact (resistance component) is Zintact, and the total impedance of Cpore (capacitance component) and Rpore (resistance component) is Zpore.

Although Rpore is necessary for the calculation of the conductance of the extraction region 501, Rpore can not be measured directly. However, from the correspondence between Rpore and Zpore, which is the total impedance of Rpore and Cpore, it is possible to calculate the conductance of the extraction region 501 by measuring Zpore.

An alternating current (I) is applied by the power source 1003 in the circuit shown in FIG. 25. Since the resistance of the voltmeter 1010 is considered to be sufficiently greater than the total impedance of Zpore and Zintact at this time, the current I flowing through the reference electrode 1009 (Rele-ref) and the voltmeter 1010 becomes smaller, such that I»i. The measurement value V measured by the voltmeter 1010 at this time is represented by equation (3) below.

$$V = Rele\text{-}ref \cdot i + Zintact \cdot I + Zpore \cdot I \quad \cdots (3)$$

(Where each element Rele-ref, Zintact, Zpore, and I in the equation are vectors of a complex plane.)

Since the first item value (Rele-refxi) is small compared to the second item value (ZintactxI) and third item value (ZporexI), the influence of the first item value on the measurement value B of the voltmeter 1010 is small compared to the second item value and third item value and can be ignored, so that equation (3) can essentially be expressed as equation (4) below.

$$V = Zintact \cdot I + Zpore \cdot I \quad \cdots (4)$$

It has been experimentally confirmed that when the frequency of the alternating current of the power source 1003 increases, the Zintact value decreases. In this experiment, two dry electrodes were positioned on the skin of four human subjects, and an alternating current was applied through the two dry electrodes. Then the frequency of the alternating current was varied, and the impedance was measured between the two dry electrodes. FIG. 26 shows a graph of the frequency characteristics of the region 502 and the dry electrodes obtained from this experiment.

Although there is large dispersion of the measurement values (impedance) of the four human subjects as shown in FIG. 26, the dispersion decreased as the frequency increased and the measurement value decreased, and it became clear that the measurement values of the four human subjects was approximately 1 kΩ when the frequency exceeded 300 Hz. It was also clear that the measurement value focused on [0] when the frequency was further increased. That is, it became clear that the influence of the impedance (Zintact) of the region 502 and the dry electrode 1002 on the measurement value V of the voltmeter 1010 could be reduced by elevating the frequency. The measurement value V from equation (4) can be expressed as equation (5) below.

$$V = Zpore \cdot I \quad \cdots (5)$$

It was experimentally confirmed that when the frequency of the alternating current of the power source 1003 is increased, the value of Zpore decreases. In the measurement system of this experiment the impedance (Zintact) of the positive electrodes (dry electrode 1002 and region 502) was set sufficiently small (less than approximately 0.5 kΩ), then the frequency of the alternating current was varied and the impedance was measured between the extraction region 501 and the positive electrode (dry electrode 1002 and region 502). FIG. 27 shows a graph of the frequency characteristics of the extraction region 501 obtained from this experiment.

As shown in FIG. 27, the impedance (Zpore) when the frequency was 8000 Hz was clearly reduced to 10 kΩ compared to the impedance (Zpore=Rpore) of 35 kΩ when the frequency was [0] (direct current) and the positive electrode resistance was 35 kΩ. Furthermore, the impedance (Zpore) when the frequency was 8000 Hz was clearly reduced to 8 kΩ and 7 kΩ compared to the impedance of 24 kΩ and 17 kΩ when the frequency was [0] (direct current) and the positive electrode resistance was 24 kΩ and 17 kΩ. That is, when the frequency increases, there is an increase in the influence of the capacitance component (Cpore) of the impedance (Zpore) of the extraction region 501, and the correlation between Zpore and Rpore clearly deteriorated.

The effect of a frequency change on measurement accuracy was determined by simulation. The dispersion of the impedance measured by the voltmeter 1010 (amount of change/average value) was determined when the negative electrode resistance (Rpore) was 5 kΩ, 10 kΩ, and 25 kΩ while varying the positive electrode capacitance component (Cintact), resistance component (Rintact), and the negative electrode capacitance component (Cpore) in this simulation. The simulation results are shown in FIGS. 28 through 30.

As shown in FIG. 28, when the negative electrode resistance (Rpore) was 5 kΩ, the measurement error was within 10% with a frequency below approximately 6000 Hz. As shown in FIG. 29, when the negative electrode resistance was 10 kΩ, the measurement error was within 10% with a frequency above approximately 200 Hz. As shown in FIG. 29, when the negative electrode resistance was 25 kΩ, the measurement error was within 10% with a frequency above approximately 100 Hz. It is clear that measurement accuracy with a measurement error within 10% can be obtained by selecting a frequency within a range greater than 100 Hz and less than 6000 Hz with the negative electrode resistance (Rpore) set greater than approximately 5 kΩ and less than approximately 25 kΩ when actually measuring the impedance (Zpore).

When the negative electrode resistance (Rpore) was 10 kΩ, the measurement error was within 5% at frequencies greater than approximately 400 Hz and less than approximately 2000 Hz; and when the negative electrode resistance was 25 kΩ, the measurement error was within 5% at frequencies greater than approximately 200 Hz and less than 800 Hz. A measurement error within 5% can be obtained when the negative electrode resistance is greater than 10 kΩ and less than 25 kΩ insofar as the frequency is greater than 400 Hz and less than 800 Hz.

That is, the impedance (Zpore) of the extraction region 501 correlates significantly with the negative electrode resistance (Rpore) by selecting frequencies greater than 100 Hz and less than 6000 Hz, which are frequencies that decrease the influence of the impedance (Zintact) of the region 502 and the dry electrode 1002, and decrease the influence of the capacitance component (Cpore) of the impedance (Zpore) of the extraction region 501.

Thus, it is possible to calculate the reciprocal of the impedance of the extraction region 501 as the conductance k of the extraction region 501 as shown in equation (6) below by applying to the skin an alternating current, and particularly an alternating current which has a frequency within a range greater than 100 Hz and less than 6000 Hz.

$$k = 1 / Rpore \propto 1 / Zpore \cdots (6)$$

Fifth Embodiment

FIG. 31 is a flow chart illustrating the analysis operation of the glucose concentration analyzer of a fifth embodiment of the present invention. The fifth embodiment differs from the fourth embodiment and is described by way of example of simultaneously measuring the glucose extraction and the impedance with reference to FIGS. 24 and 31. Since the structure of the glucose concentration analyzer of the fifth embodiment is identical to that of the fourth embodiment, further description is omitted.

In the fifth embodiment, the operation sin steps S51 and S52 of FIG. 31 are identical to the operations in step S41 and S42 (refer to FIG. 24) of the fourth embodiment. Preprocessing (skin permeability facilitation treatment) and the like is also identical to that of the fourth embodiment.

In step S53 of the fifth embodiment, a current composed of an alternating current overlaid on a direct current of 80 μA±30 μA (500 Hz) is applied to the skin. Glucose is thus extracted from the extraction region 501 by the direct current component of the current applied to the skin 500. The impedance of the extraction region 501 can be obtained based on the measurement value measured by the voltmeter 1010 through the current applied to the skin 500.

In step S54, the amount of extracted glucose is measured by the glucose sensor 1007, and the control and analysis part 1008 calculates the glucose extraction rate J based on the amount of extracted glucose. The conductance k of the extraction region 501 can be calculated based on the impedance of the extraction region 501.

In step S55, the glucose concentration (blood sugar level C) is calculated based on the conductance k of the extraction region 501 and the glucose extraction rate J similar to step S7 of the fourth embodiment.

In the fifth embodiment, tissue fluid can be extracted by a direct current at the same time the impedance is obtained by an alternating current so as to calculate the conductance of the skin by applying a current composed of overlaid alternating current and direct current. The total time of the conductance measurement operation and the tissue fluid extraction operation is therefore reduced.

The other effects of the fifth embodiment are identical to the effects of the fourth embodiment.

Sixth Embodiment

FIG. 32 is a block diagram showing the structure of a glucose concentration analyzer of the sixth embodiment of the present invention. The sixth embodiment differs from the fourth embodiment, and is described by way of example in which the conductance measuring apparatus which calculates the conductance is separate from the concentration analyzer which calculates the glucose concentration. The structure of the glucose concentration analyzer 1200 of the sixth embodiment of the present invention is first described below referring to FIG. 32.

The glucose concentration analyzer 1200 of the sixth embodiment is configured by a conductance measuring apparatus 1200a which is mounted on the skin 500 of a subject to calculate the conductance of the extraction region 501a and extract glucose to a mesh sheet 1004, and a concentration analyzer 1200b which calculates the glucose extraction rate from the mesh sheet 1004 that contains glucose, and calculates the concentration of the glucose (blood-sugar level) from the glucose extraction rate and the conductance calculated by the conductance measuring apparatus 1200a.

Since the structure of the conductance measuring apparatus 1200a is identical to the structure of the glucose concentration analyzer 1000 of the fourth embodiment with the exception that the sensor member 1005 and the glucose sensor 1007 are omitted, the description is abbreviated.

The concentration analyzer 1200b is provided with a receiving part 1201 for receiving the mesh sheet 1004 which contains the glucose obtained in the conductance measuring apparatus 1200a, a glucose sensor 1202 for measuring the amount of glucose extracted from the mesh part 1004, a control and analyzing part 1203 for calculating the glucose extraction rate from the amount of extracted glucose obtained by the glucose sensor 1202, an input part 1204 configured by buttons or the like for starting the analysis by the concentration analyzer 1200b, and a display part 1205 for displaying analysis results and the like. A sensor member 1206 which functions similar to the sensor member 1005 of the fourth embodiment is installed when the glucose sensor 1202measures the amount of extracted glucose from the mesh sheet 1004.

The control and analyzing part 1203 is connected by wire or wirelessly to the control and analyzing part 1008 of the conductance measuring apparatus 1200a, and is capable of receiving the conductance value and the current application time of the direct current applied to extract the tissue fluid from the control and analyzing part 1008 of the conductance measuring apparatus 1200a.

FIGS. 33 and 34 are flow charts illustrating the operation of the concentration analyzer and the conductance measuring apparatus of the glucose concentration analyzer of the sixth embodiment of the present invention. The analysis operation performed by the glucose concentration analyzer 1200 of the sixth embodiment of the present invention is described below with reference to FIGS. 2, 3, and 32 through 34.

When the blood-sugar level is measured using the glucose concentration analyzer 1200 of the sixth embodiment, extraction holes 501a (refer to FIG. 3) are first formed in the skin 500 of the subject using the needle roller 600 (refer to FIG. 2).

The subject also installs the mesh sheet 1004 in the conductance measuring apparatus 1200a of the glucose concentration analyzer 1200. In step S61 of FIG. 33 in the conductance measuring apparatus 1200a, a sensor (not shown in the drawing) determines whether or not the mesh sheet 1004 has been installed. This determination is repeated when the mesh sheet 1004 has not been installed. The routine continues to step S62 when the mesh sheet 1004 has been installed.

Thereafter, the conductance measuring apparatus 1200a is mounted on the skin 500 of the subject so that the negative electrode (mesh sheet 1004) is in contact with the extraction region 501 in which extraction holes 501a have been formed. The subject subsequently issues a measurement start instruction by pressing the button (input part 1011) of the conductance measuring apparatus 1200a.

The conductance measuring apparatus 1200a also determines whether or not a measurement start instruction has been issued in step S62. This determination is repeated when a measurement start instruction has not been issued. When a measurement start instruction has been issued, a direct current is applied to the skin 500 in step S63 similar to step S3 of the fourth embodiment. Thus, glucose is extracted to the mesh sheet 1004.

In the subsequent steps S64 and S65 in the conductance measuring apparatus 1200a, an alternating current is applied to the skin and the conductance of the extraction region 501 is calculated similar to steps S5 and S6 of the fourth embodiment.

In the following step S66, the conductance measuring apparatus 1200a sends the conductance of the extraction region 501 calculated in step S65 from the control and analyzing part 1008 of the conductance measuring apparatus 1200a to the control and analyzing part 1203 of the concentration analyzer 1200b.

The subject then removes the mesh sheet 1004 from the conductance measuring apparatus 1200a, and installs the mesh sheet 1004 in the concentration analyzer 1200b.

In step S71 in the concentration analyzer 1200b, a determination is made as to whether not the mesh sheet 1004 has been installed. This determination is repeated when a mesh sheet 1004 has not been installed. The routine continues to step S72 when the mesh sheet 1004 has been installed.

The subject installs the sensor member 1206 in the concentration analyzer 1200b after the mesh sheet 1004 has been installed in the concentration analyzer 1200b. The concentration analyzer 1200b determines at this time whether or not a sensor member 1206 is installed in step S72. This determination is repeated when the sensor member 1206 has not been installed. When a sensor member 1206 has been installed the routine continues to step S73.

The subject issues a measurement start instruction by operating the input part 1204, which is configured as a button or the like, after the mesh sheet 1004 and the sensor member 1206 have been installed in the concentration analyzer 1200b. In the concentration analyzer 1200b, a determination is also made in step S73 as to whether or not a measurement start instruction has been issued. This determination is repeated when a measurement start instruction has not been issued. When a measurement start instruction has been issued, the control and analysis part 1203 calculates the glucose extraction amount in step S74 based on the signals output from the glucose sensor 1202 and the photodiode similar to step S4 of the fourth embodiment.

In step S75, a determination is then made in the concentration analyzer 1200b as to whether or not information such as the conductance of the extraction region 501 has been received from the conductance measuring apparatus 1200a. This determination is repeated when information has not been received. When information has been received the routine continues to step S76. The information of conductance and the like includes the application time of the direct current applied to extract the glucose in step S63.

In step S76, the glucose concentration (blood sugar level) is calculated in the concentration analyzer 1200b. That is, the control and analysis part 1203 calculates the glucose extraction rate J by dividing the amount of extracted glucose obtained in step S74 by the application time of the direct current received in step S75. The control and analysis part 1203 then calculates the glucose concentration (blood sugar level C) of the tissue fluid based on the conductance k of the extraction region 501 and the glucose extraction rate J.

In the sixth embodiment described above, it is possible to prevent substances such as glucose oxidase, peroxidase, and color-producing agent contained in the sensor member 1206, which are required to measure the amount of extracted glucose, from coming into contact with the skin of the subject by separating the conductance measuring apparatus 1200a which measures conductance and extracts glucose and measures conductance, and the concentration analyzer 1200b which is provided with the glucose sensor 1202 that obtains the amount of extracted glucose while mounted on the skin of the subject. The safety of the subject s therefore increased.

The other effects of the sixth embodiment are identical to the effects of the fourth embodiment.

The embodiments and examples in this disclosure are only examples in all aspects, and are not to be considered limiting in any way. The scope of the present invention is defined by the scope of the claims and not by the embodiment or description of the embodiments, and includes all modifications within the scope of the claims and the meanings and equivalences therein.

For example, although a NaCl concentration of 0 mM was used in the first through third embodiment, the present invention is not limited to this use inasmuch as physiological saline solution which has a low conductivity and a NaCl concentration less than 5 mM may also be used.

Although examples are described in which the presence of ions such as Na+ and the like are localized by waiting a predetermined time after the glucose has been extracted in the first through third embodiment, the present invention is not limited to this mode inasmuch as active mixing may also accomplished by pipetting or stirring or the like. Thus, the ions may be made uniform more rapidly.

Although the first through third embodiments are described by way of examples in which tissue fluid which contains glucose is extracted through the extraction region 501 in which micropores (extraction holes 501a) have been formed by the needle roller 600 (skin permeability facilitating treatment), the present invention is not limited to this mode inasmuch as tissue fluid may also be extracted from the extraction region without forming micropores (no skin permeability facilitating treatment).

Although the first through third embodiment are described by way of examples in which the extraction of tissue fluid containing glucose is facilitated by applying a direct current to the skin, the present invention is not limited to this mode inasmuch as glucose also may be extracted by passive diffusion without applying a direct current to the skin. The correlation between conductance and skin permeability can be improved by using purified water as the medium for holding the tissue fluid even when extraction is by passive diffusion.

Although a mesh sheet which has a thickness of approximately 50 μm is used in the first and second embodiments, the present invention is not limited to this configuration insofar as the thickness of the mesh sheet is less than 500 μm.

Although the first, second and fourth through sixth embodiments are described by way of examples in which the frequency of the alternating current of the power source is approximately 500 Hz, the present invention is not limited to this frequency inasmuch as a frequency other than 500 Hz may also be used. 500 Frequencies greater than 100 Hz and less than 6000 Hz are examples of such frequencies other than 500 Hz. Measurement errors can be reduced by suitably selecting the frequency.

Although the fourth through sixth embodiments have been described by ways of example in which purified water is included in the mesh sheet as a substance for holding glucose, the present invention is not limited to this mode inasmuch as a conductive medium such as physiological saline solution may also be used as the holding substance.

Although the impedance of the negative electrode is determined in the above embodiments by controlling the current of the power source and measuring the voltage between the negative electrode and positive electrode, the present invention is not limited to this mode inasmuch as the impedance of the negative electrode may also be determined by controlling the voltage of the power source and measuring the current between the negative electrode and positive electrode.

Although micropores are formed in the skin by a needle roller in the above embodiments, the present invention is not limited to this mode inasmuch as passive diffusion may be promoted by ultrasonically irradiating the extraction region of the skin to lower the barrier function of the skin, or alcohol and surface active agent may be applied as enhancers to promote transdermal movement of tissue fluid at the extraction region of the skin. The barrier function of the skin may also be lowered by irradiating the skin with a laser.

Although the examples of the above embodiments use a mesh sheet as a medium containing part or tissue fluid holding part, the present invention is not limited to this mode inasmuch as a sheet-like piece of paper may also be used as a medium containing part, or a non-sheet-like piece of paper or nylon or the like may be used. Furthermore, a collecting medium configured by a nonconductive gel such as polyacrylic acid or the like adhered to a plate-like member may also be used. It is desirable that the medium containing part is sheet-like from the perspective of being close to the electrode and skin. The analyte extraction speed can be increased by being having the electrode close to the skin.

Although the embodiments have been described by way of examples in which the present invention is applied to a glucose concentration analyzer that extracts glucose from a living body and calculates the blood sugar level, the present invention is not limited to this mode inasmuch as the present invention may also be applied to a component concentration analyzer that analyzes the concentration of analytes other than glucose extracted from a living body. The analytes extracted by the component concentration analyzer to which the present invention can be applied may be, for example, a biochemical component, or drug administered to the subject or the like. Examples of biochemical components include proteins such as albumin, globulin, enzymes and the like which are one type of biochemical component. Examples of biochemical components other than proteins include creatinine, creatine, uric acid, amino acid, fructose, galactose, pentose, glycogen, lactic acid, pyruvic acid, ketone bodies and the like. Examples of drugs include digitalis, theophylline, arrhythmia drug, anti-epileptic drug, amino glycoside antibiotic, glycopeptide antibiotic, antithrombic agent, immunosuppressant and the like.

The glucose concentration analyzer of the above embodiments, or a component concentration analyzer incorporating the present invention to extract analytes other than glucose from a living body, may also utilize a control and analysis part and the like to analyze proteins and non-protein biochemical components and drugs using measurement method other than HPLC (high performance liquid chromatography).

Although the above embodiment shave been described by way of examples in which extraction holes 501a are formed in the skin 500 of a subject using a needle roller 600, the present invention is not limited to this mode inasmuch as a modified puncture tool 700 shown in FIGS. 21 and 22 may also be used. The puncture tool 700 is a device which forms extraction holes (micropores) in the skin of a living body to extract body fluid by installing a sterilized microneedle chip 800 (refer to FIG. 22) and bringing the microneedles 810 of the microneedle chip 800 against the skin of the living body (for example, the skin of a human body). More specifically, the puncture tool 700 is provided with a spring to exert force on a piston, and a locking mechanism to lock a state in which the spring exerts a force on the piston. When using puncture tool 700, the puncture tool 700 is set on the skin of the user with the microneedle chip 800 installed in the bottom part of the piston. When the locking mechanism is subsequently released by pressing a button 710, the microneedle chip 800 impacts the skin through the elastic force of the spring. Thus, forming micropores.

The foregoing detailed description and examples have been provided by way of explanation and illustration, and are not intended to limit the scope of the appended claims. Many variations in the presently preferred embodiments will be obvious to one of ordinary skill in the art, and remain within the scope of the appended claims and their equivalents.

## Claims

**1.** A method of measuring skin conductance, comprising the steps of:

supplying a low conductivity medium as a medium for holding tissue fluid that contains a predetermined component of a subject to a medium containing part;
disposing the medium containing part on an extraction region of a skin of the subject;
extracting the tissue fluid through the extraction region into the low conductivity medium within the medium containing part;
supplying electric power between a first electrode for supplying electric power to the extraction region and a second electrode for supplying electric power to the skin outside the extraction region; and
measuring the conductance of the extraction region based on the electric power supplied between the first electrode and second electrode.

**2.** The method of measuring skin conductance according to claim 1, wherein the resistivity of the low conductivity medium in the medium containing part is 1.67 KΩ•cm or greater when the extraction of the tissue fluid begins.

**3.** The method of measuring skin conductance according to claim 1 or 2, wherein the low conductivity medium is purified water.

**4.** A method of analyzing a component concentration, comprising the steps of:

supplying a low conductivity medium as a medium for holding tissue fluid that contains a predetermined component of a subject to a medium containing part;
disposing the medium containing part on an extraction region of the skin of the subject;
extracting the tissue fluid through the extraction region into the low conductivity medium within the medium containing part;
supplying electric power between a first electrode for supplying electric power to the extraction region and a second electrode for supplying electric power to the skin outside the extraction region;
measuring a value that corresponds to the amount of the predetermined component contained in the tissue fluid extracted through the extraction region into the low conductivity medium; and
calculating the concentration of the predetermined component in the tissue fluid based on a energization result of the electric power supplied between the first electrode and the second electrode, and the value obtained at the measuring step.

**5.** The method of analyzing a component concentration according to claim 4, wherein the calculating step is performed so as to measure a conductance of the extraction region as the energization result.

**6.** The method of analyzing a component concentration according to claim 5, wherein the conductance is measured when the ions in the low conductivity medium are in a substantially homogeneous condition after the extracting step.

**7.** A skin conductance measuring apparatus, comprising:

a medium containing part, desposed on an extraction region of the skin of a subject, to accommodate a low conductivity medium for holding tissue fluid that contains a predetermined component of the subject;
a first electrode for supplying electric power to the extraction region;
a second electrode for supplying electric power to a first region outside of the extraction region of the skin of the subject;
a power source for supplying electric power to the first electrode and the second electrode; and
a conductance measuring part for measuring the conductance of the extraction region based on the electric power supplied from the power source to the first electrode and the second electrode.

**8.** A component concentration analyzer, comprising:

a medium containing part, desposed on an extraction region of the skin of a subject, to accommodate a low conductivity medium for holding tissue fluid that contains a predetermined component of the subject;
a first electrode for supplying electric power to the extraction region;
a second electrode for supplying electric power to the skin outside the extraction region;
a power source for supplying electric power to the first electrode and the second electrode;
a energization result measuring part for measuring a energization result by supplying electric power from the power source to the first electrode and second electrode;
a component amount measuring part for measuring a value corresponding to the amount of a predetermined component contained in the tissue fluid extracted through the extraction region into a low conductivity medium in the medium containing part; and
an analyzing part for calculating the concentration of the predetermined component in the tissue fluid based on the energization result and the value corresponding to the amount of the predetermined component.

**9.** A skin conductance measuring apparatus, comprising:

a first electrode for supplying electric power to a position corresponding to an extraction region which is a skin of a subject performed a skin permeability facilitating treatment for extracting tissue fluid that contains a predetermined component;
a second electrode for supplying electric power to a position corresponding to a region which is the skin of the subject outside the extraction region;
a power source for applying an alternating current as electric power to the skin of the subject through the first electrode and the second electrode;
an electrical information obtaining part for obtaining electrical information by applying the alternating current from the power source to the skin of the subject; and
a calculating part for calculating the conductance of the skin of the subject based on the electrical information.

**10.** The skin conductance measuring apparatus according to claim 9, further comprising a tissue fluid retention part for retaining the tissue fluid.

**11.** The skin conductance measuring apparatus according to claim 9 or 10, wherein the power source is capable of applying a direct current to the skin of the subject through the first electrode and the second electrode.

**12.** The skin conductance measuring apparatus according to claim 9 or 10, wherein the power source is capable of applying a current composed of an overlaid alternating current and direct current to the skin of the subject through the first electrode and the second electrode..

**13.** The skin conductance measuring apparatus according to claim 10, wherein the tissue fluid retention part includes a retention substance for retaining the tissue fluid.

**14.** The skin conductance measuring apparatus according to claim 13, wherein the retention substance is a non-conductive medium.

**15.** The skin conductance measuring apparatus according to any one of claims 9 to 14, wherein the extraction region is the skin of the subject in which a micropore is formed as the skin permeability facilitating treatment.

**16.** The skin conductance measuring apparatus according to any one of claims 9 to 15, wherein the frequency of the alternating current is greater than 100 Hz and less than 6000 Hz.

**17.** The skin conductance measuring apparatus according to any one of claims 9 to 16, wherein the conductance of the skin includes the conductance of the extraction region.

**18.** The skin conductance measuring apparatus according to any one of claims 9 to 17, wherein electrical information includes the impedance of the extraction region.

**19.** The skin conductance measuring apparatus according to any one of claims 9 to 18, wherein the predetermined component includes glucose.

**20.** The skin conductance measuring apparatus according to any one of claims 9 to 19, further comprising a reference electrode for measuring a voltage applied between the second electrode and the extraction region.

**21.** A component concentration analyzer, comprising:

a first electrode for supplying electric power to a position corresponding to an extraction region which is a skin of a subject performed a skin permeability facilitating treatment for extracting tissue fluid that contains a predetermined component;
a second electrode for supplying electric power to a position corresponding to a region which is the skin of the subject outside the extraction region;
a tissue fluid retention part for retaining the tissue fluid extracted from the extraction region.
a power source for applying an alternating current as electric power to the skin of the subject through the first electrode and second electrode;
an electrical information obtaining part for obtaining electrical information that is obtained by applying an alternating current from the power source to the skin of the subject;
a calculating part for calculating the conductance of the skin of the subject based on the electrical information;
a component amount obtaining part for obtaining a value corresponding to the amount of the predetermined component in the tissue fluid retention part; and
a component concentration obtaining part for calculating the concentration of the predetermined component in the tissue fluid based on the value obtained by the component amount obtaining part, and the conductance of the extraction region calculated by the calculating part.

**22.** A method of measuring skin conductance, comprising the steps of:

performing a skin permeability facilitating treatment on the extraction region of the skin of a subject; and
calculating the conductance of the skin based on electrical information obtained by applying an alternating current to the extraction region and a region outside the extraction region of the skin of the subject.

**23.** A method of analyzing a component concentration, comprising the steps of:

performing a skin permeability facilitating treatment on the extraction region of the skin of a subject;
extracting tissue fluid which contains a predetermined component through the extraction region of the skin of the subject;
obtaining a value corresponding to an amount of the predetermined component of the tissue fluid extracted by the extracting step;
calculating a conductance of the skin based on electrical information obtained by applying an alternating current to the extraction region and a region outside the extraction region of the skin of the subject; and
analyzing the concentration of the predetermined component contained in the tissue fluid of the subject based on the value obtained by the obtaining step and the conductance of the skin obtained by the calculating step.

**24.** The method of analyzing a component concentration of claim 23, wherein the extracting step and the calculating step are a step of calculating the conductance of the skin based on electrical information obtained using an alternating current while extracting tissue fluid which contains the predetermined component through the extraction region of the skin of the subject using a direct current, by applying a current composed of an overlaid alternating current and direct current to the skin of the subject.

FIG. 1

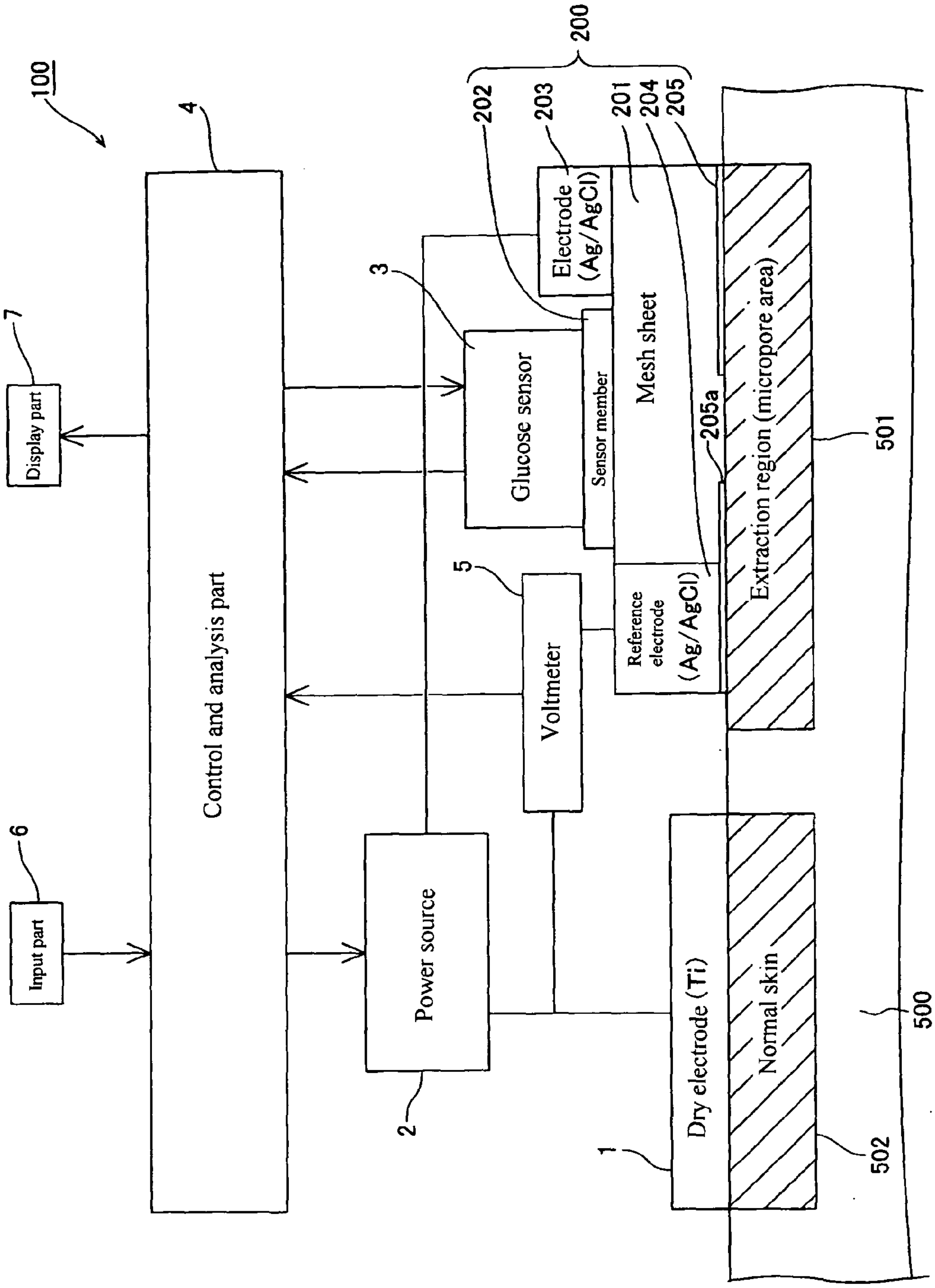
100
4
Control and analysis part
7
Display part
6
Input part
3
Glucose sensor
Sensor member
202
203
Electrode (Ag/AgCl)
200
201
204
205
Mesh sheet
205a
Extraction region (micropore area)
501
5
Voltmeter
Reference electrode (Ag/AgCl)
Power source
2
Dry electrode (Ti)
Normal skin
1
502
500

FIG. 2

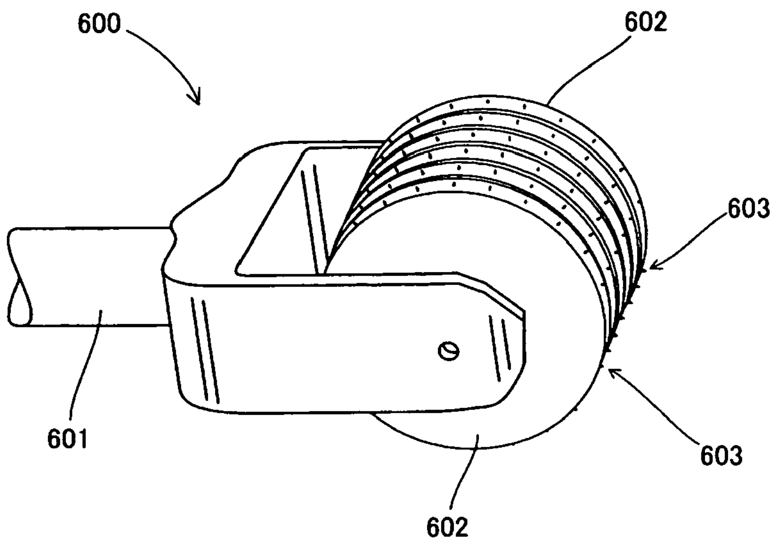

FIG. 3

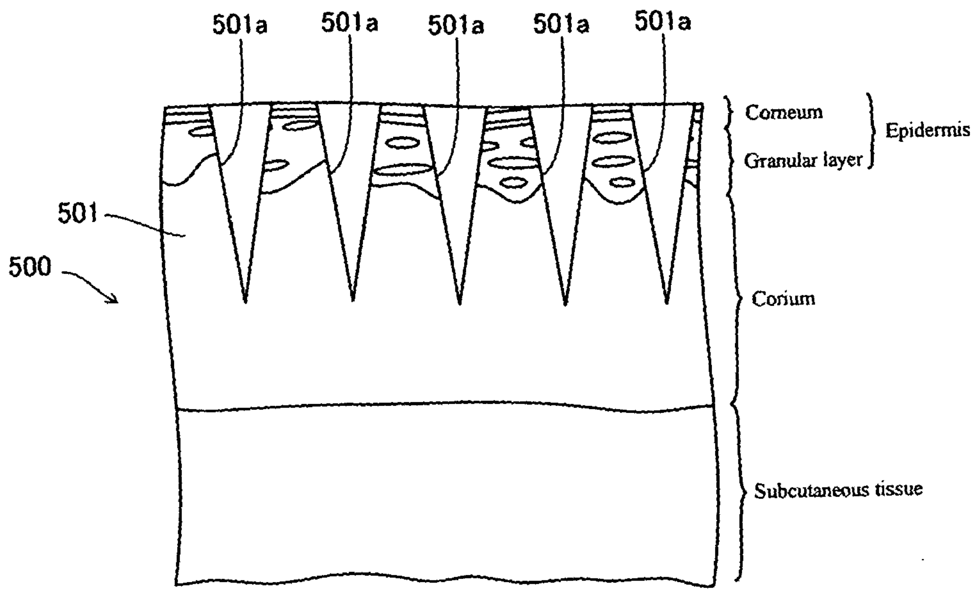
501a
501a
501a
501a
501a
Corneum
Granular layer
Epidermis
501
500
Corium
Subcutaneous tissue

FIG. 4

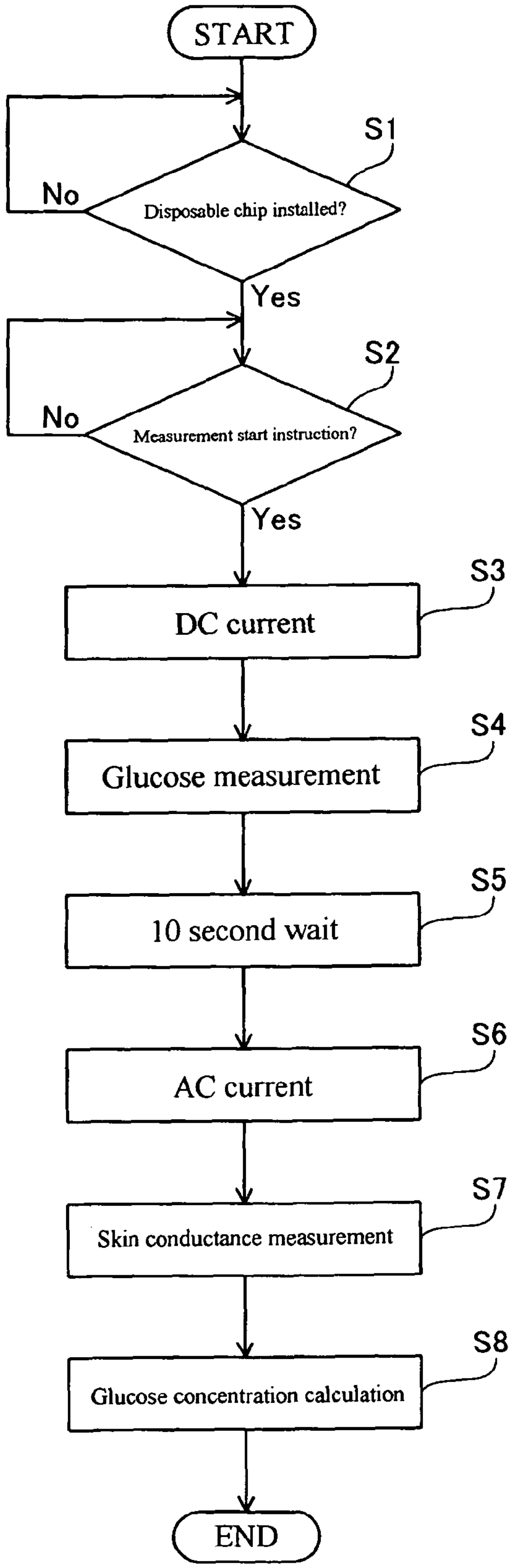
START
S1
No
Disposable chip installed?
Yes
S2
No
Measurement start instruction?
Yes
S3
DC current
S4
Glucose measurement
S5
10 second wait
S6
AC current
S7
Skin conductance measurement
S8
Glucose concentration calculation
END

# FIG. 5

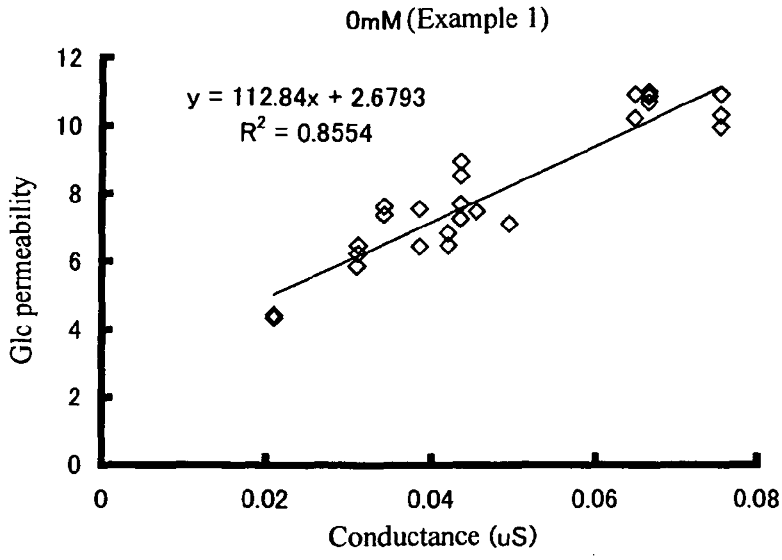
0mM (Example 1)
y = 112.84x + 2.6793
R² = 0.8554
Glc permeability
12
10
8
6
4
2
0
0
0.02
0.04
0.06
0.08
Conductance (uS)

# FIG. 6

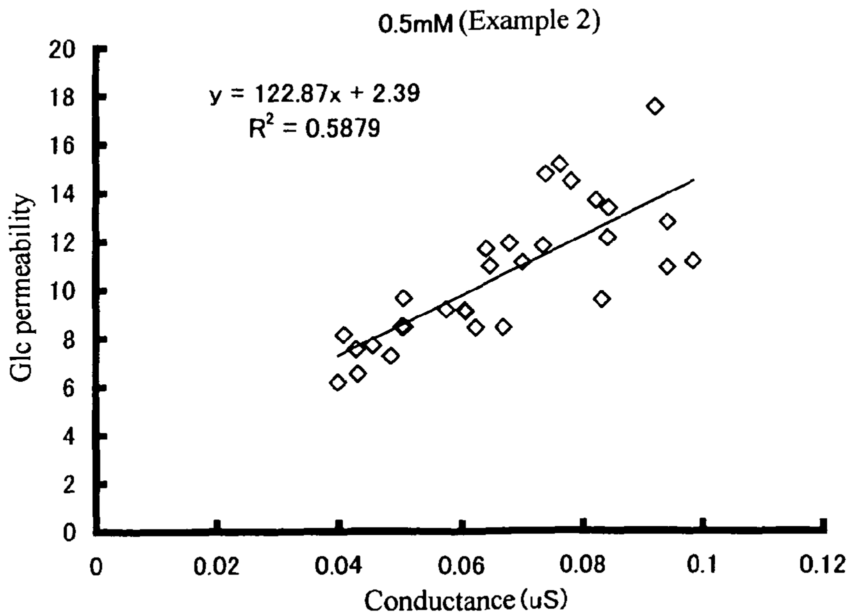
0.5mM (Example 2)
y = 122.87x + 2.39
R² = 0.5879
Glc permeability
20
18
16
14
12
10
8
6
4
2
0
0
0.02
0.04
0.06
0.08
0.1
0.12
Conductance (uS)

FIG. 7

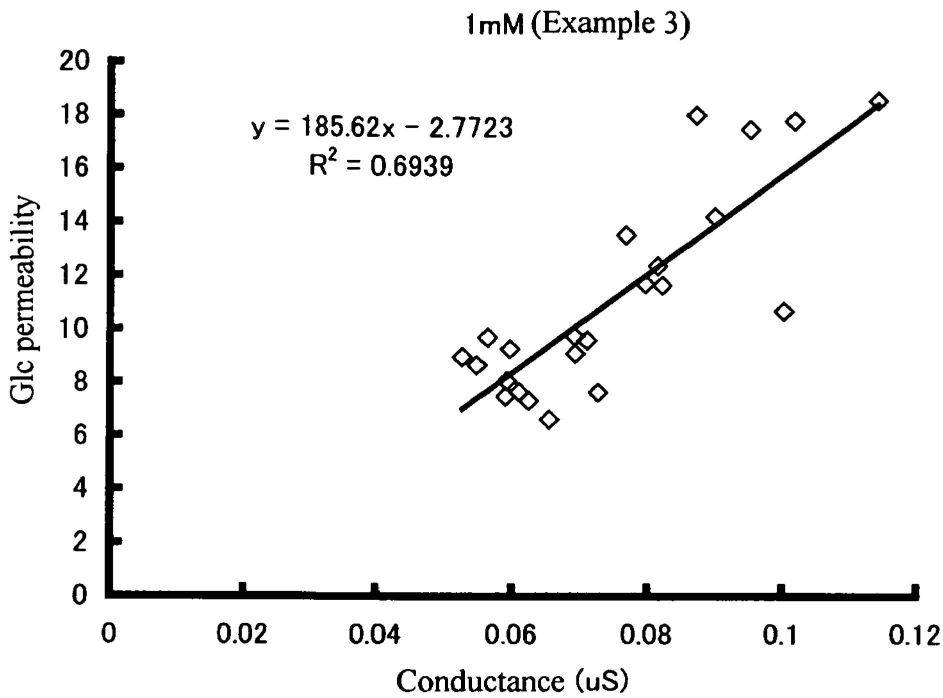
1mM (Example 3)
y = 185.62x - 2.7723
R² = 0.6939
Glc permeability
20
18
16
14
12
10
8
6
4
2
0
0
0.02
0.04
0.06
0.08
0.1
0.12
Conductance (uS)

FIG. 8

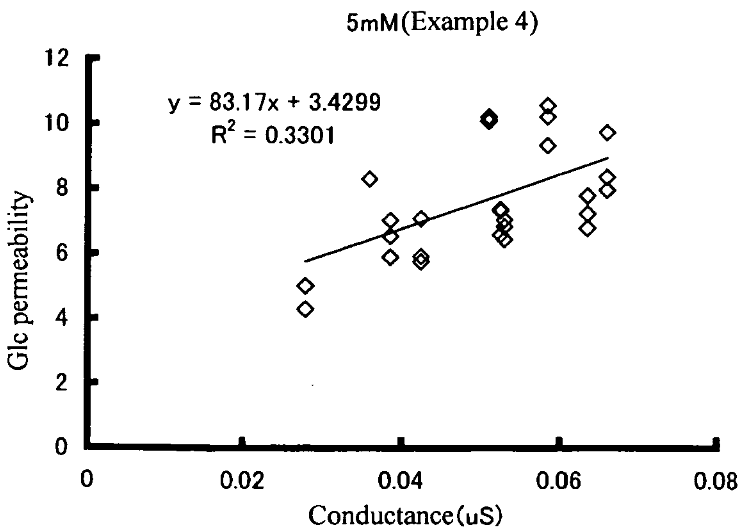
5mM(Example 4)
y = 83.17x + 3.4299
R² = 0.3301
Glc permeability
12
10
8
6
4
2
0
0
0.02
0.04
0.06
0.08
Conductance(uS)

# FIG. 9

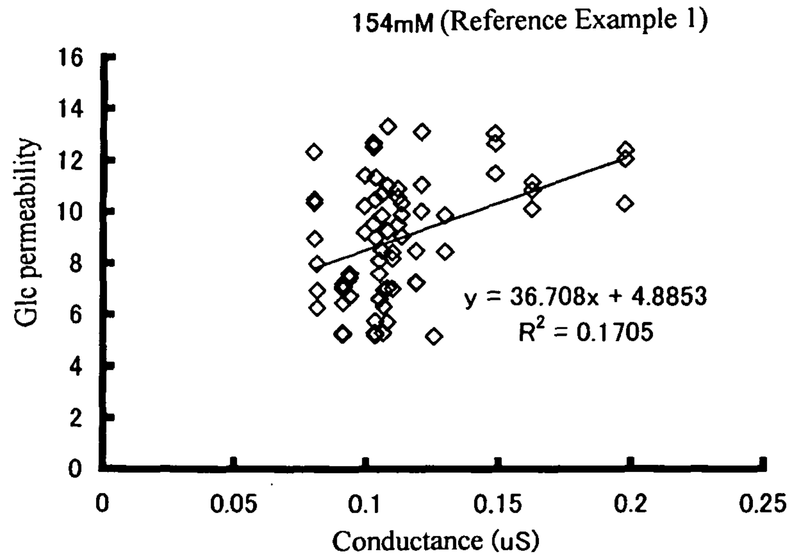
154mM (Reference Example 1)
Glc permeability
16
14
12
10
8
6
4
2
0
0
0.05
0.1
0.15
0.2
0.25
Conductance (uS)
y = 36.708x + 4.8853
$R^2$ = 0.1705

# FIG. 10

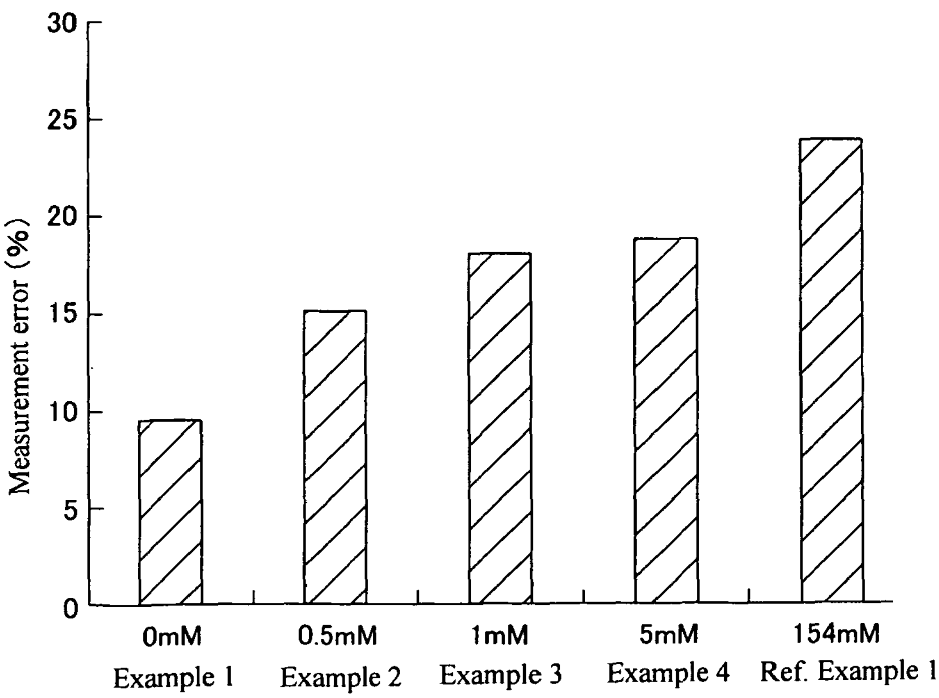
Measurement error (%)
30
25
20
15
10
5
0
0mM
Example 1
0.5mM
Example 2
1mM
Example 3
5mM
Example 4
154mM
Ref. Example 1

# FIG. 11

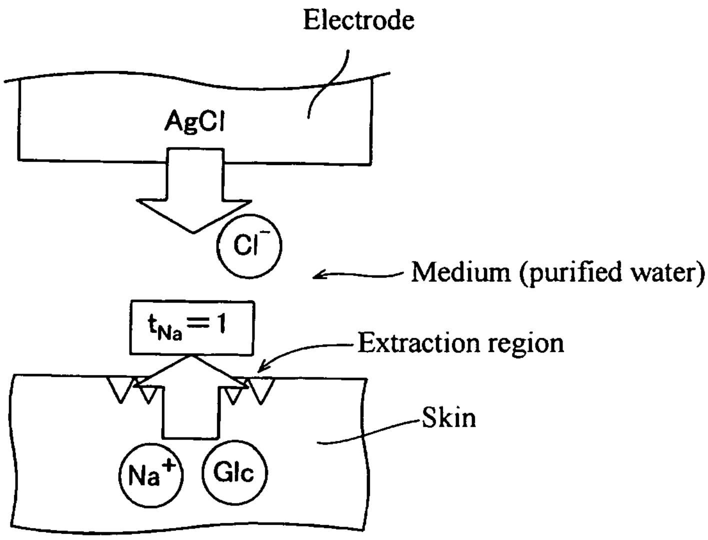
Electrode
AgCl
Cl⁻
Medium (purified water)
$t_{Na}=1$
Extraction region
Skin
Na⁺
Glc

# FIG. 12

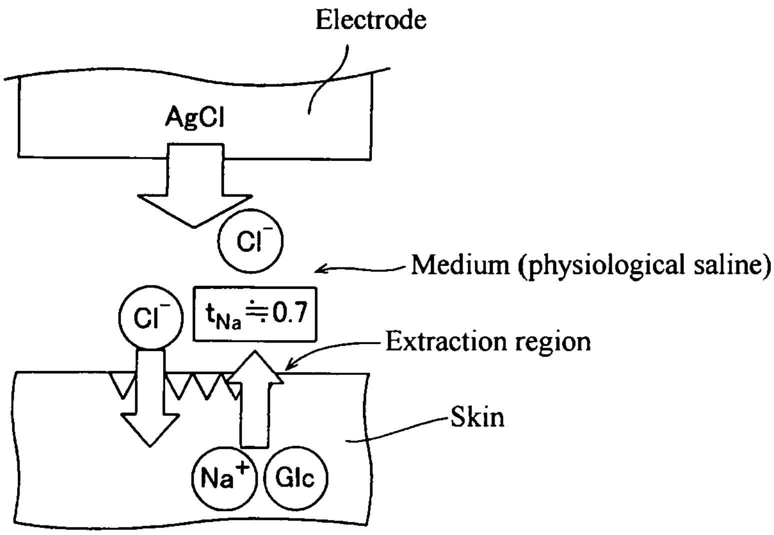
Electrode
AgCl
Cl⁻
Medium (physiological saline)
Cl⁻
$t_{Na}\fallingdotseq 0.7$
Extraction region
Skin
Na⁺
Glc

FIG. 13

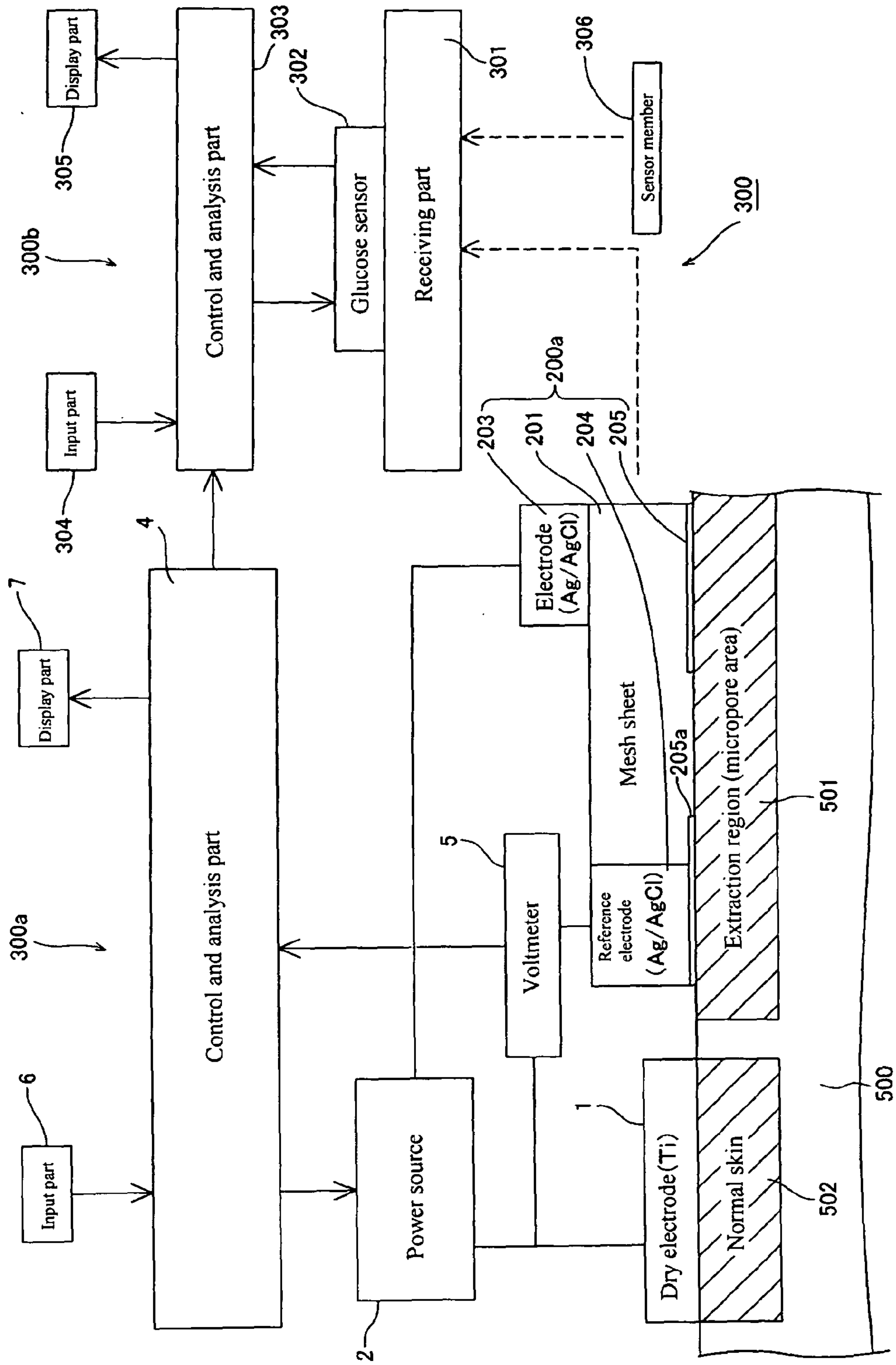

Input part
6
Control and analysis part
Display part
7
4
Power source
2
300a
Voltmeter
5
Dry electrode (Ti)
1
Normal skin
502
500
Reference electrode (Ag/AgCl)
Extraction region (micropore area)
501
Mesh sheet
205a
Electrode (Ag/AgCl)
203
201
204
205
200a
Input part
304
Control and analysis part
303
Display part
305
300b
Glucose sensor
302
Receiving part
301
Sensor member
306
300

# FIG. 14

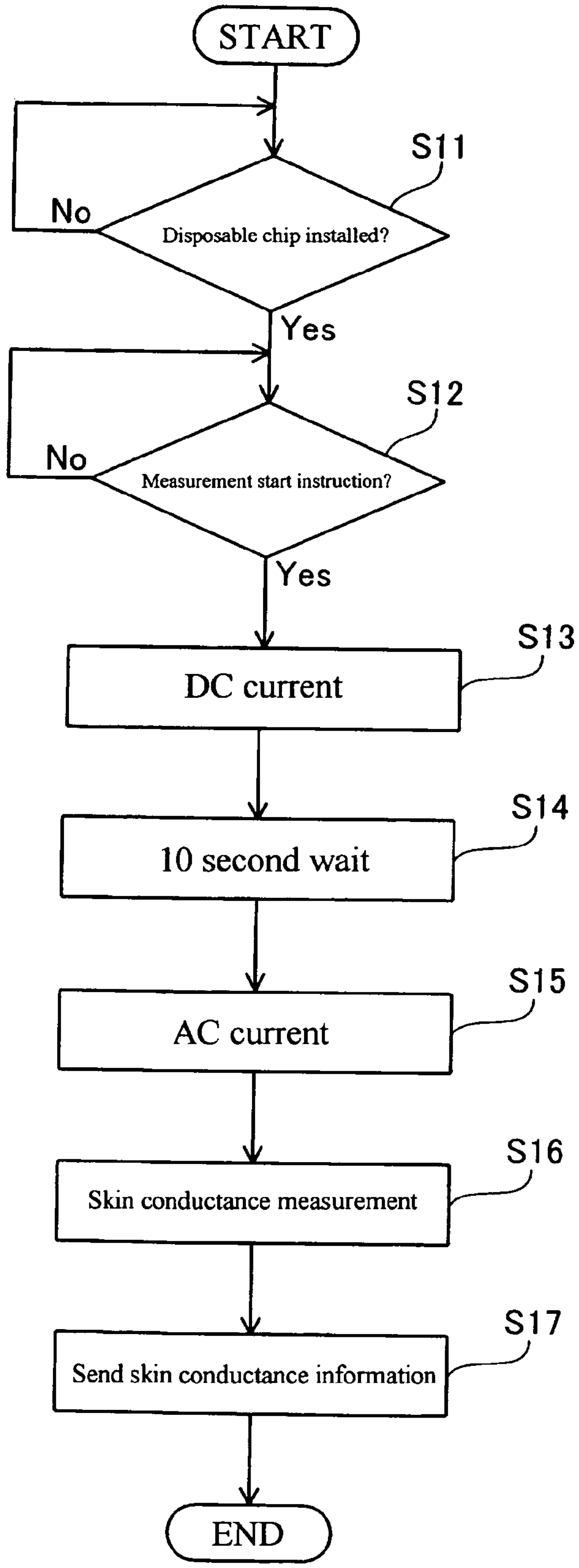
START
S11
No
Disposable chip installed?
Yes
S12
No
Measurement start instruction?
Yes
S13
DC current
S14
10 second wait
S15
AC current
S16
Skin conductance measurement
S17
Send skin conductance information
END

FIG. 15

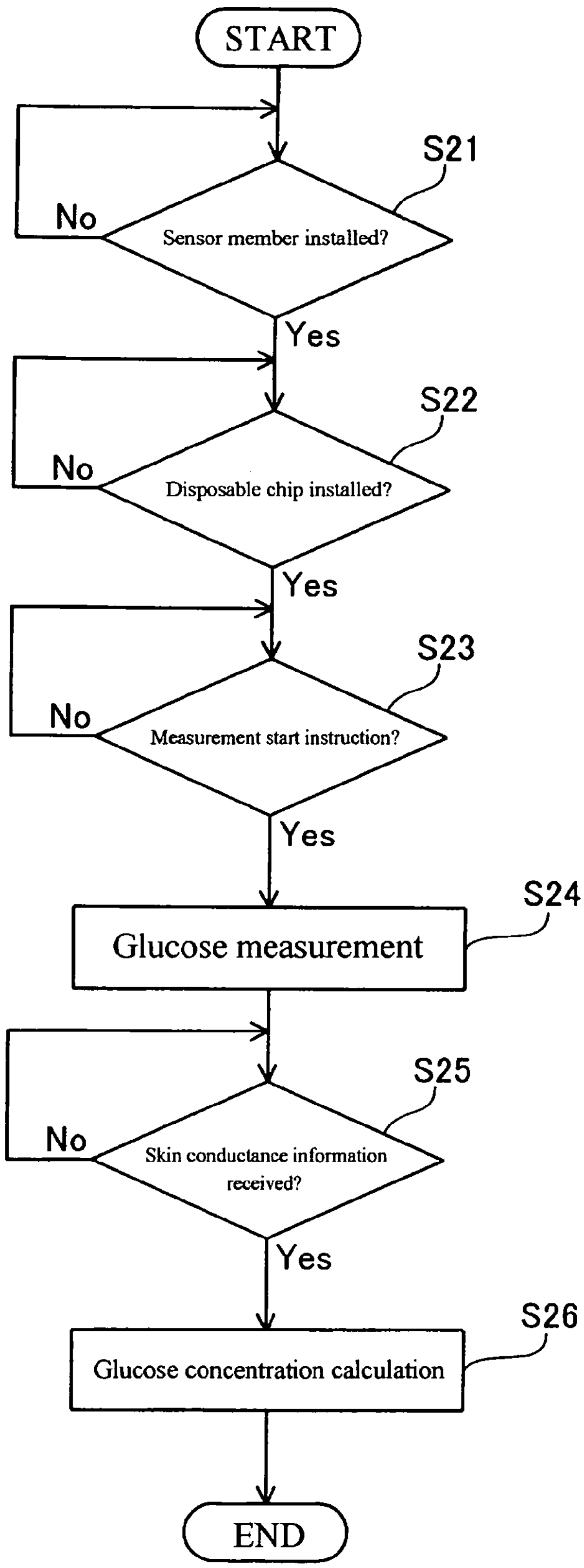
START
S21
Sensor member installed?
No
Yes
S22
Disposable chip installed?
No
Yes
S23
Measurement start instruction?
No
Yes
S24
Glucose measurement
S25
Skin conductance information received?
No
Yes
S26
Glucose concentration calculation
END

FIG. 16

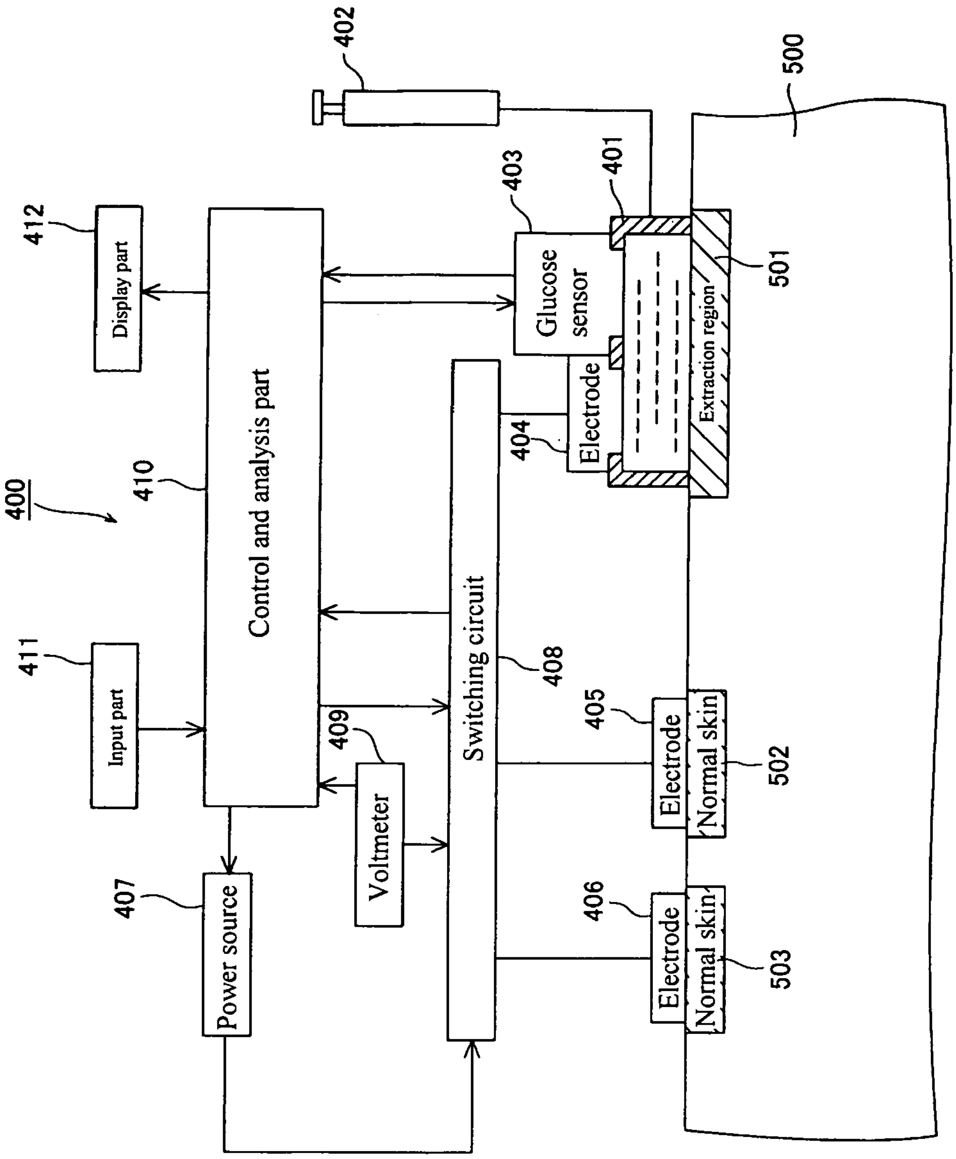
400
402
412
Display part
410
Control and analysis part
403
Glucose sensor
401
404
Electrode
Extraction region
501
500
411
Input part
Switching circuit
408
409
Voltmeter
405
Electrode
Normal skin
502
407
Power source
406
Electrode
Normal skin
503

# FIG. 17

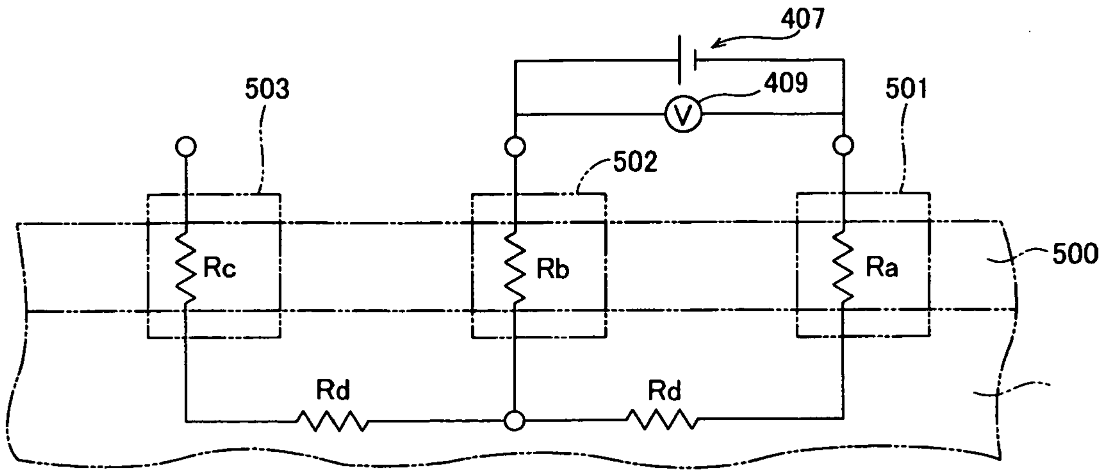
407
409
503
501
502
Rc
Rb
Ra
500
Rd
Rd

# FIG. 18

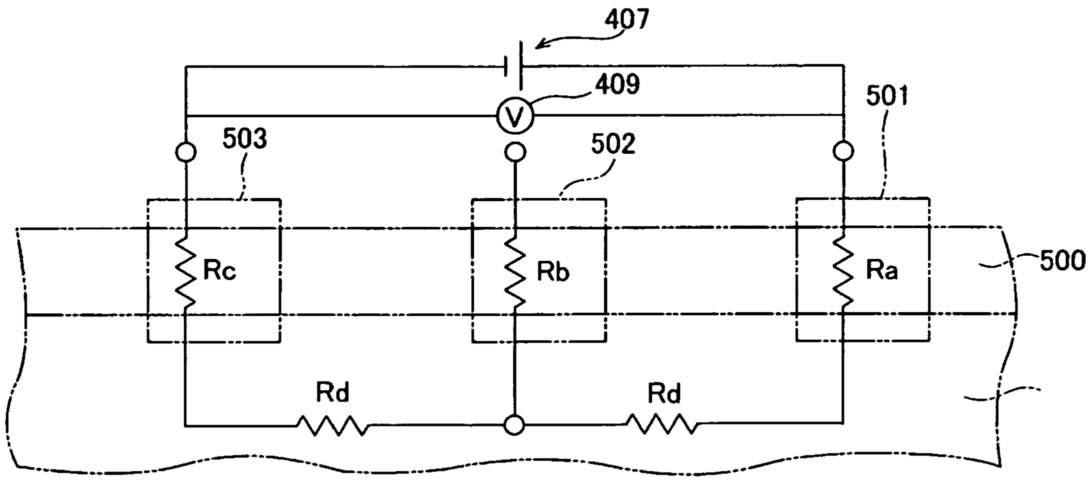
407
409
501
503
502
Rc
Rb
Ra
500
Rd
Rd

FIG. 19

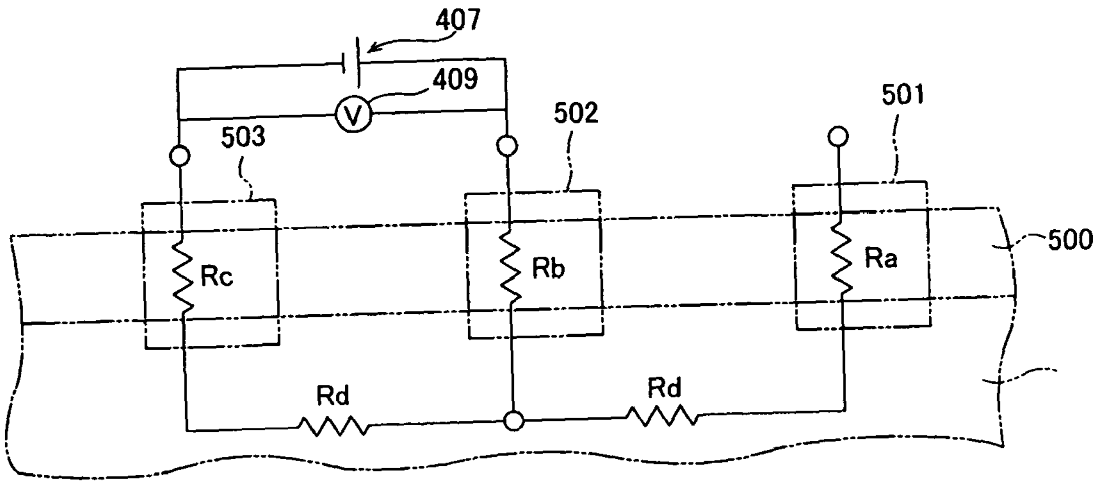

FIG. 20

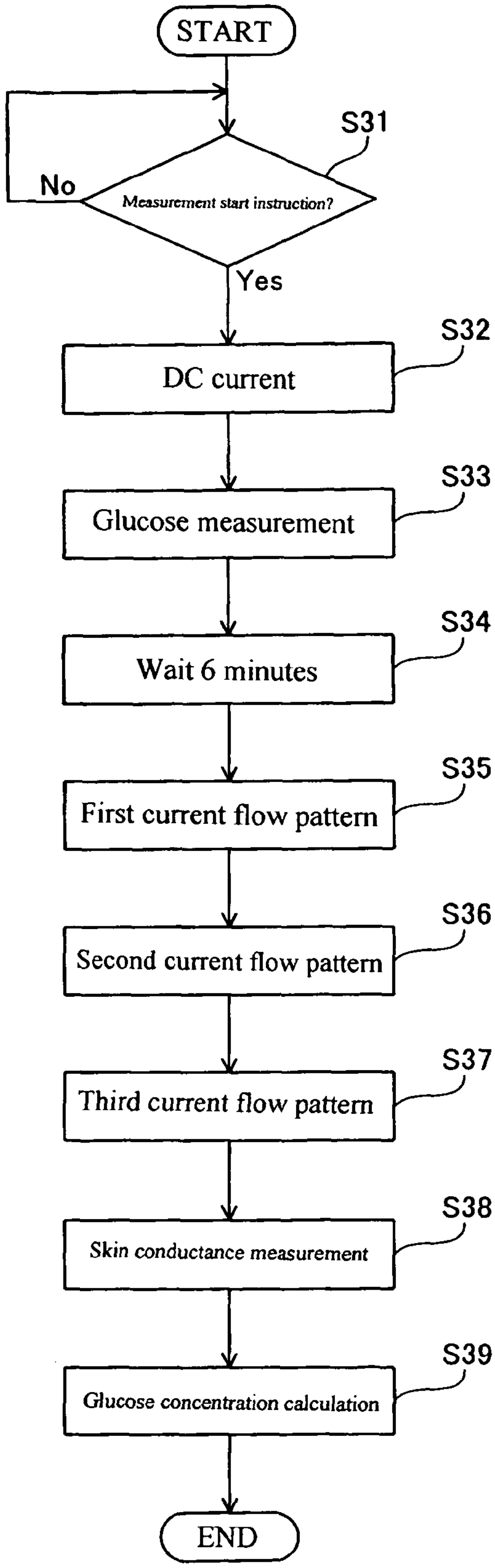
START
S31
Measurement start instruction?
No
Yes
S32
DC current
S33
Glucose measurement
S34
Wait 6 minutes
S35
First current flow pattern
S36
Second current flow pattern
S37
Third current flow pattern
S38
Skin conductance measurement
S39
Glucose concentration calculation
END

FIG. 21

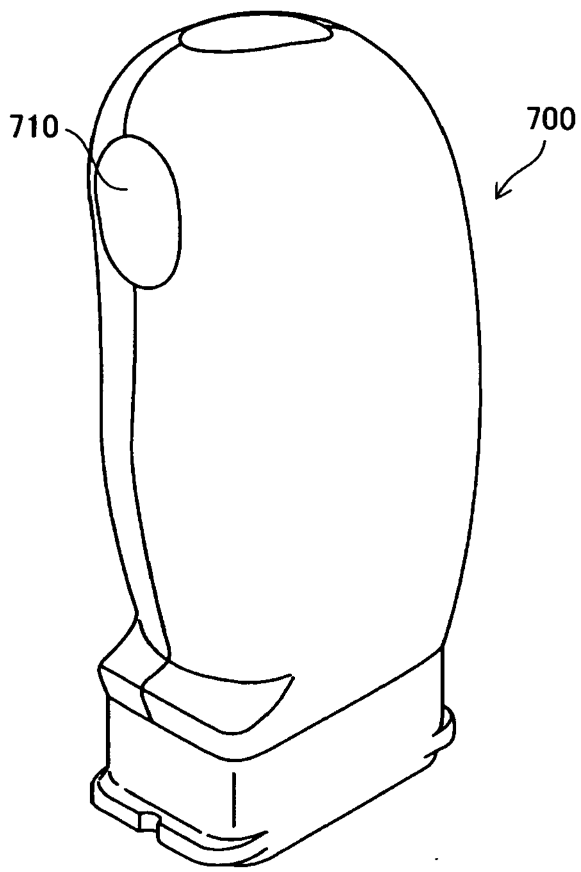

FIG. 22

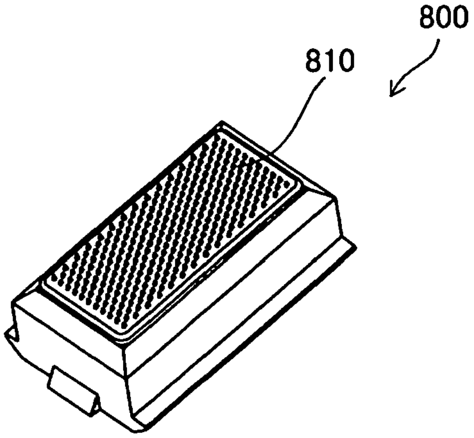

FIG. 23

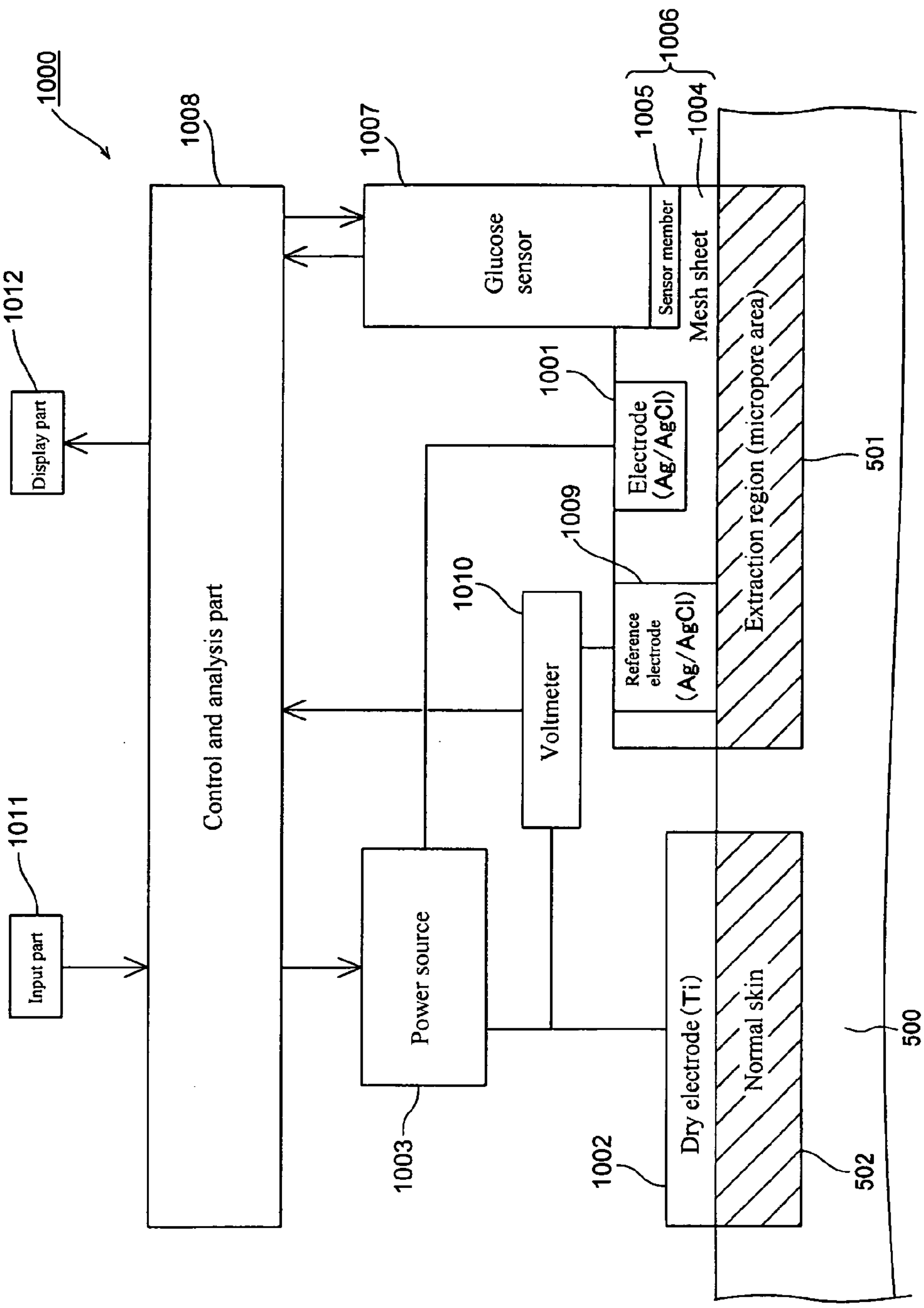
1000
Input part
1011
Display part
1012
Control and analysis part
1008
Power source
1003
Voltmeter
1010
Glucose sensor
1007
Sensor member
1005
Mesh sheet
1004
1006
Electrode (Ag/AgCl)
1001
Reference electrode (Ag/AgCl)
1009
Dry electrode (Ti)
1002
Extraction region (micropore area)
501
Normal skin
502
500

FIG. 24

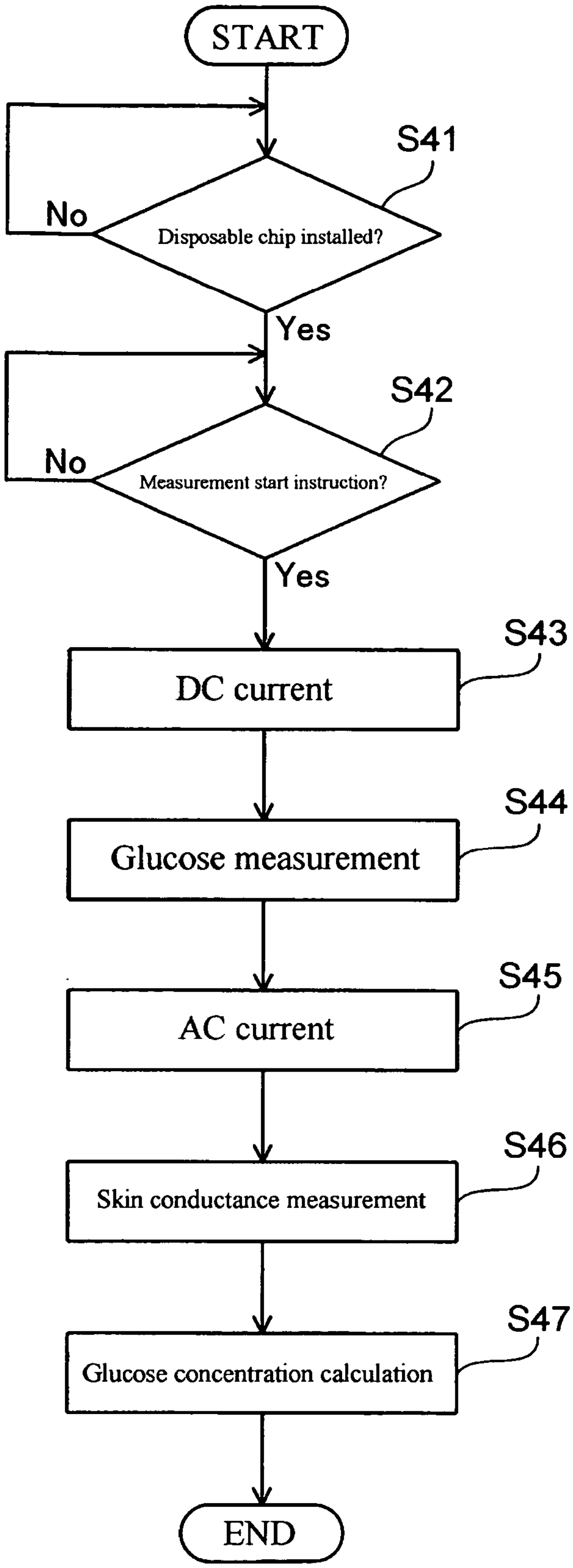
START
S41
No
Disposable chip installed?
Yes
S42
No
Measurement start instruction?
Yes
S43
DC current
S44
Glucose measurement
S45
AC current
S46
Skin conductance measurement
S47
Glucose concentration calculation
END

# FIG. 25

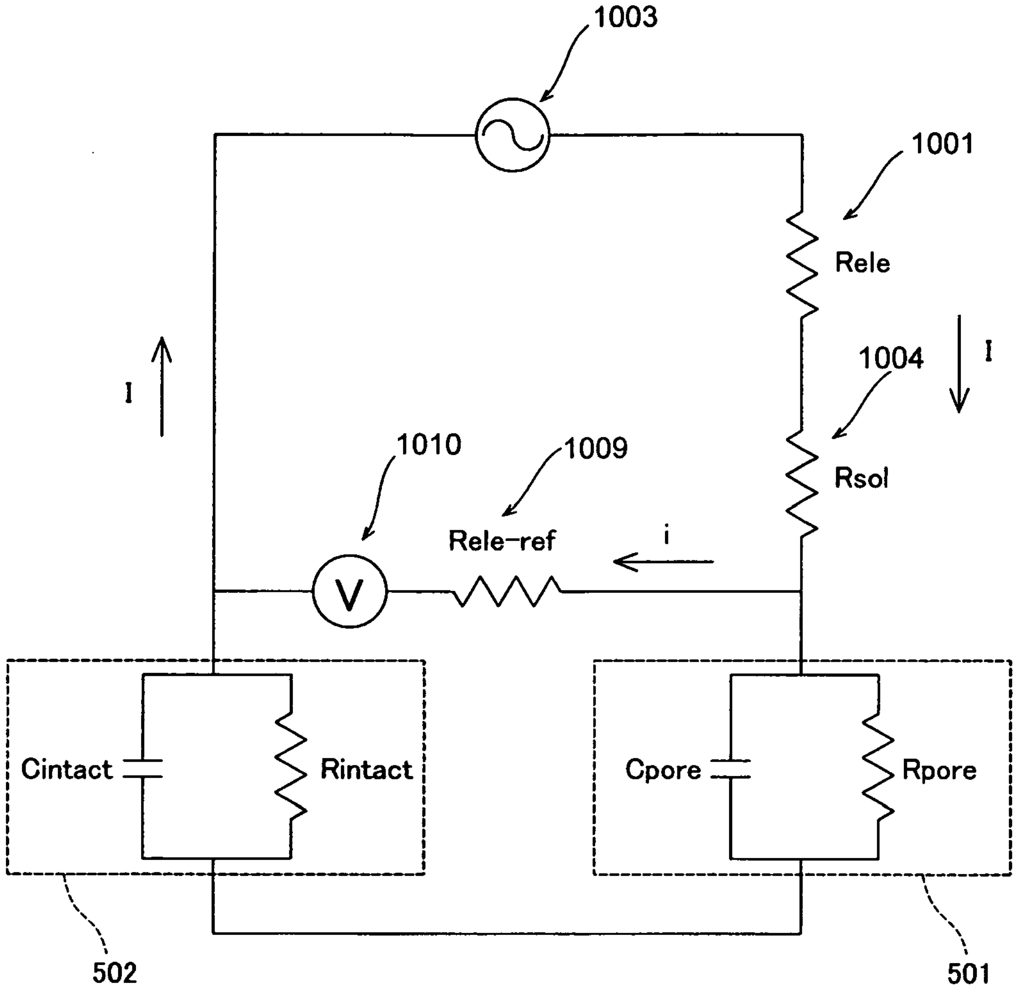
1003
1001
Rele
I
1004
I
Rsol
1010
1009
Rele-ref
i
V
Cintact
Rintact
Cpore
Rpore
502
501

# FIG. 26

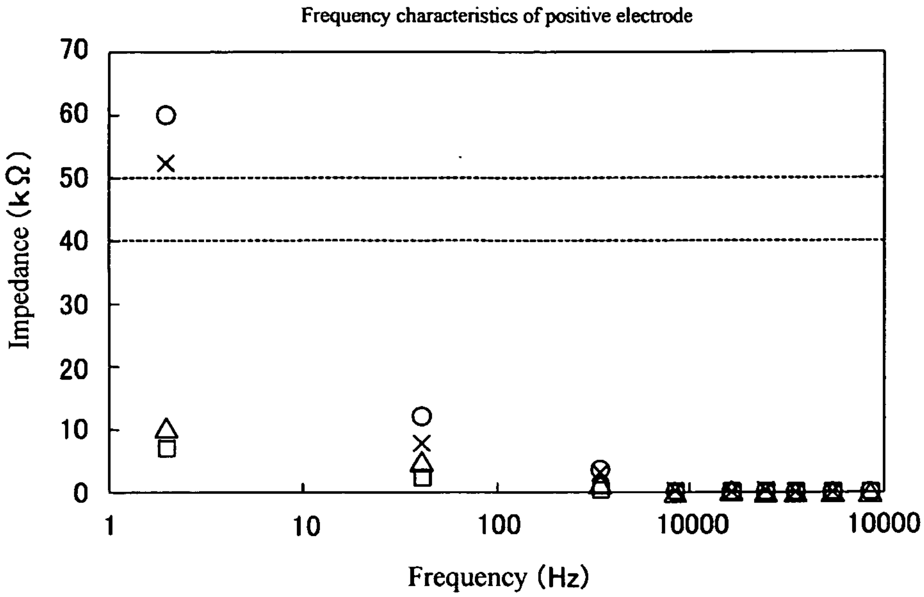
Frequency characteristics of positive electrode
70
60
50
40
30
20
10
0
Impedance (kΩ)
1
10
100
10000
10000
Frequency (Hz)

# FIG. 27

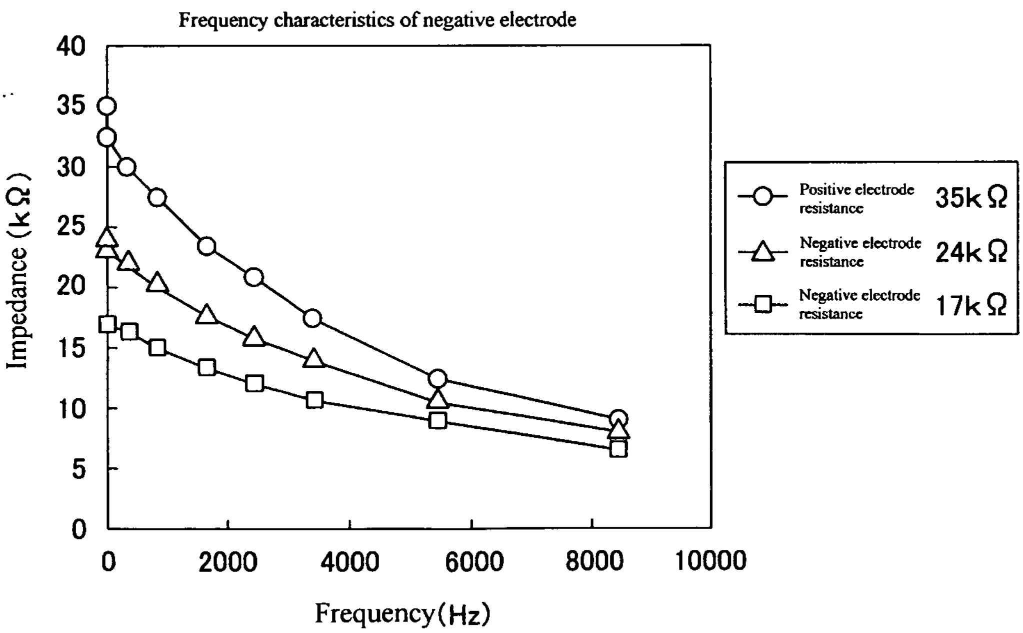
Frequency characteristics of negative electrode
40
35
30
25
20
15
10
5
0
Impedance (kΩ)
0
2000
4000
6000
8000
10000
Frequency(Hz)
Positive electrode resistance 35kΩ
Negative electrode resistance 24kΩ
Negative electrode resistance 17kΩ

# FIG. 28

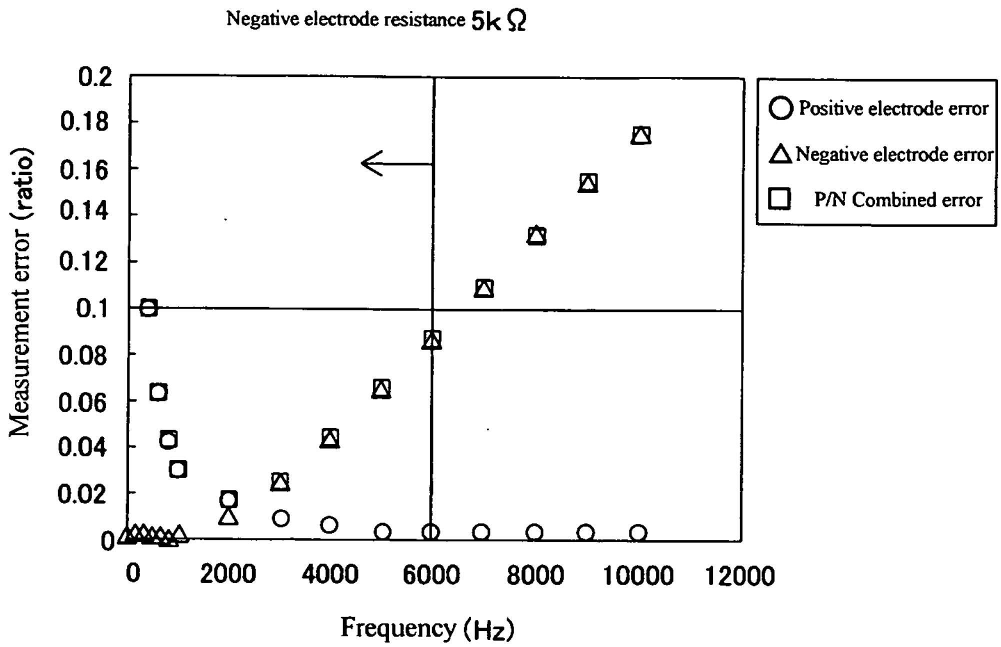
Negative electrode resistance 5k Ω
Measurement error (ratio)
0.2
0.18
0.16
0.14
0.12
0.1
0.08
0.06
0.04
0.02
0
0
2000
4000
6000
8000
10000
12000
Frequency (Hz)
Positive electrode error
Negative electrode error
P/N Combined error

# FIG. 29

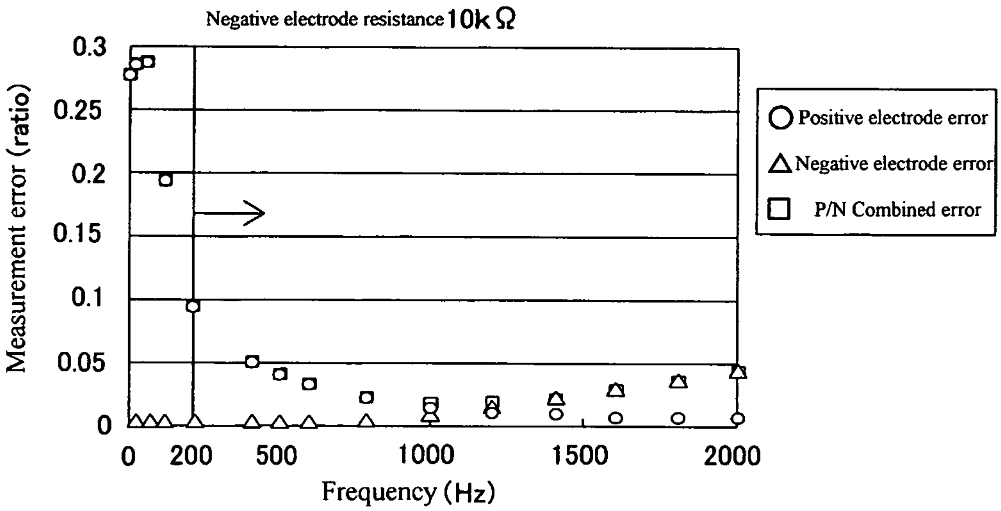
Negative electrode resistance 10k Ω
Measurement error (ratio)
0.3
0.25
0.2
0.15
0.1
0.05
0
0
200
500
1000
1500
2000
Frequency (Hz)
Positive electrode error
Negative electrode error
P/N Combined error

# FIG. 30

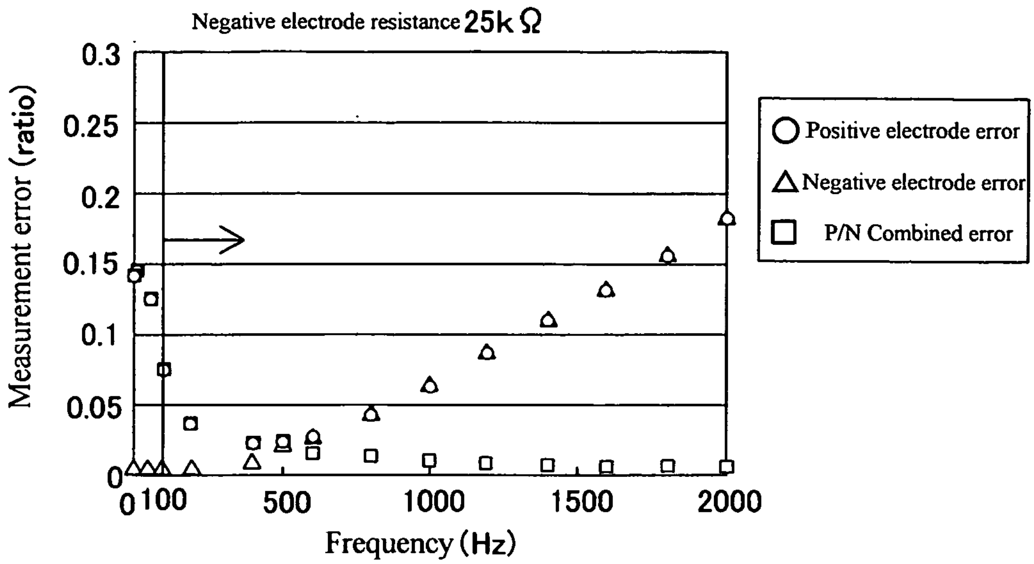
Negative electrode resistance 25kΩ
0.3
0.25
0.2
0.15
0.1
0.05
0
Measurement error (ratio)
0
100
500
1000
1500
2000
Frequency (Hz)
Positive electrode error
Negative electrode error
P/N Combined error

# FIG. 31

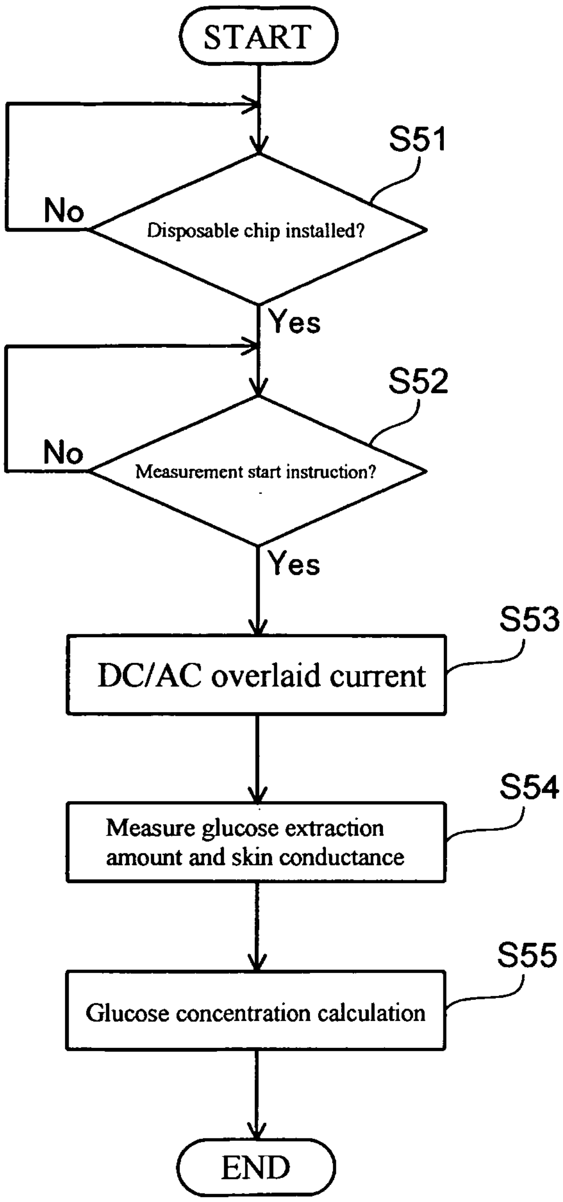
START
S51
No
Disposable chip installed?
Yes
S52
No
Measurement start instruction?
Yes
S53
DC/AC overlaid current
S54
Measure glucose extraction amount and skin conductance
S55
Glucose concentration calculation
END

FIG. 32

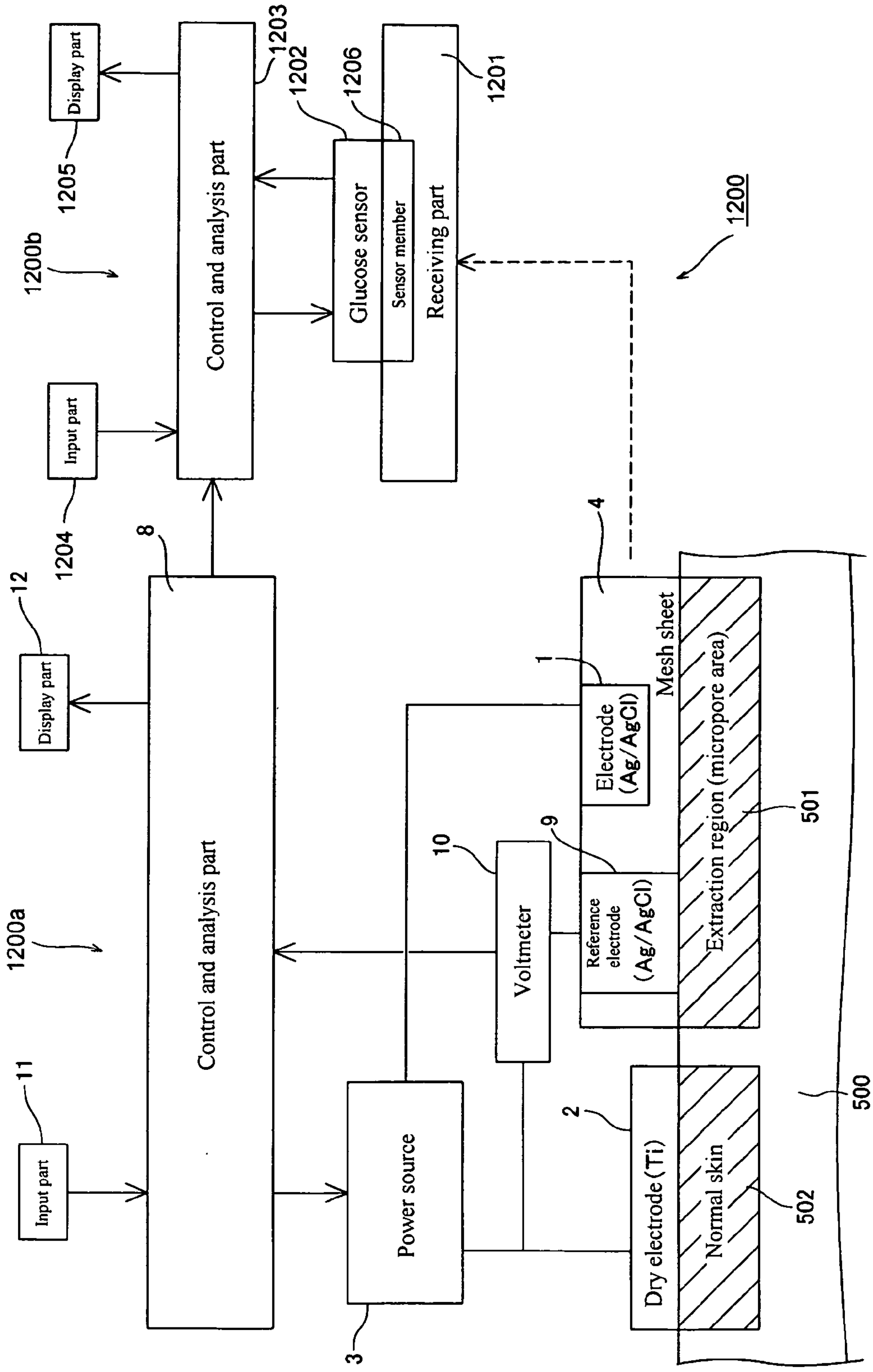
1200b
Display part
1205
Control and analysis part
1203
1202
1206
1201
Input part
1204
Glucose sensor
Sensor member
Receiving part
1200
1200a
Input part
11
Display part
12
8
Control and analysis part
Power source
3
Voltmeter
10
Reference electrode (Ag/AgCl)
9
Electrode (Ag/AgCl)
1
Mesh sheet
4
Dry electrode (Ti)
2
Extraction region (micropore area)
501
Normal skin
502
500

FIG. 33

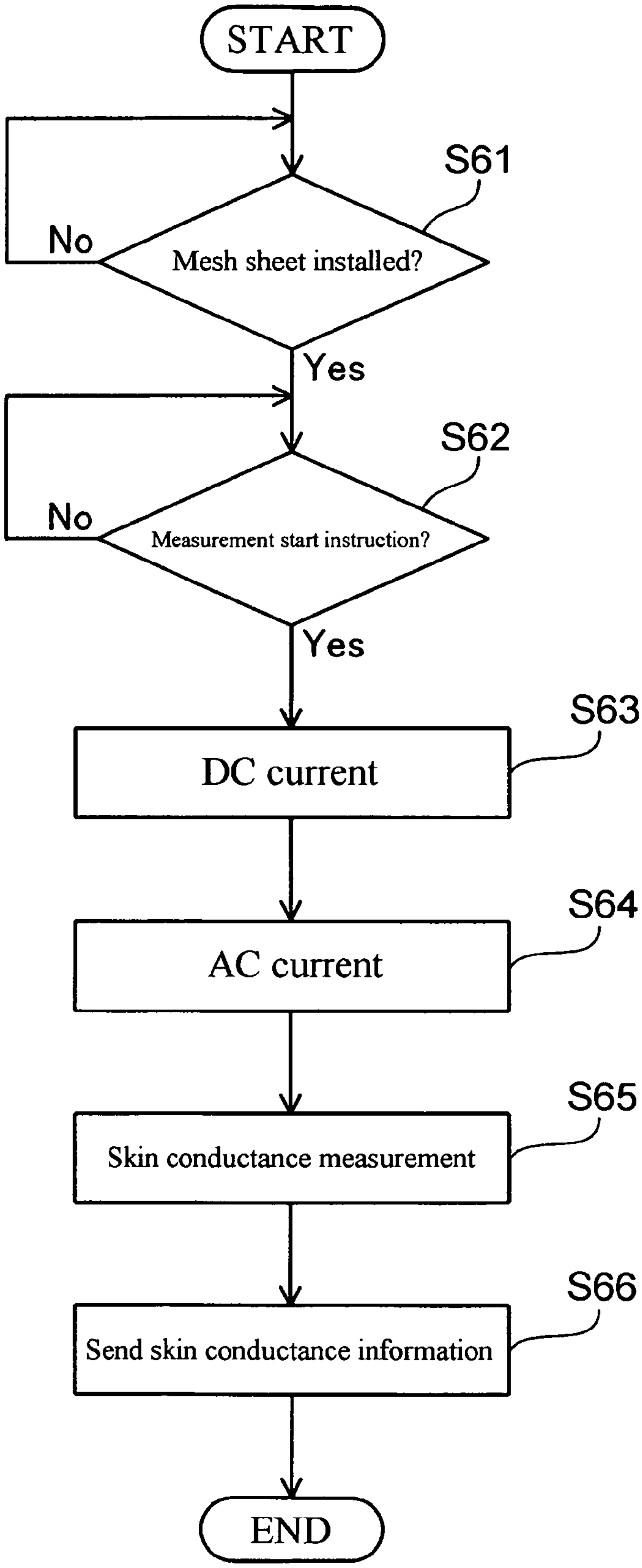
START
S61
No
Mesh sheet installed?
Yes
S62
No
Measurement start instruction?
Yes
S63
DC current
S64
AC current
S65
Skin conductance measurement
S66
Send skin conductance information
END

FIG. 34

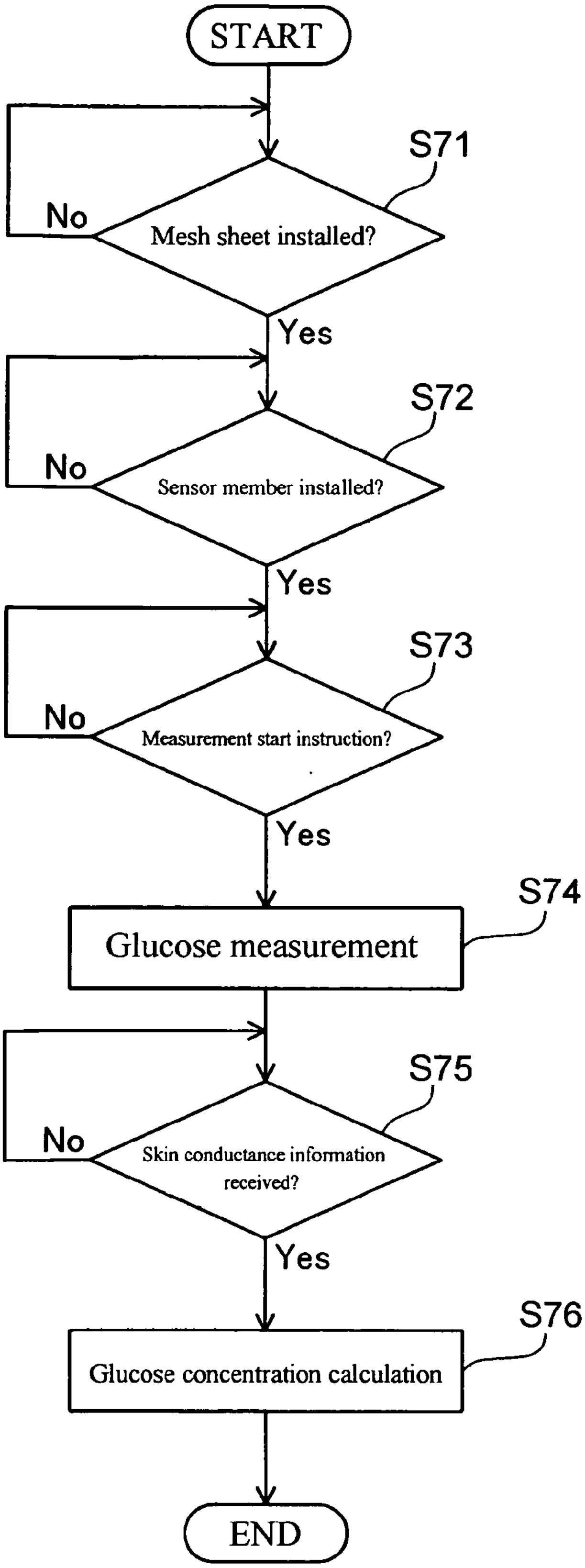
START
S71
No
Mesh sheet installed?
Yes
S72
No
Sensor member installed?
Yes
S73
No
Measurement start instruction?
Yes
S74
Glucose measurement
S75
No
Skin conductance information received?
Yes
S76
Glucose concentration calculation
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060029991 A
- US 2006009991 A